# EUROPEAN PATENT APPLICATION

(11) **EP 4 230 204 A1**
(43) Date of publication of application: **23.08.2023**
(21) Application number: 21883179.0
(22) Date of filing: 19.10.2021
(51) Int. Cl.: A61K 31/4184, A61K 31/496, A61P 35/00

(54) **THIOBENZIMIDAZOLE DERIVATIVE OR PHARMACEUTICALLY ACCEPTABLE SALT THEREOF AND USE THEREOF**

(30) Priority: 19.10.2020 KR 20200135175; 18.10.2021 KR 20210138673
(71) Applicant: Korea University Research and Business Foundation, Seoul 02841 (KR)
(72) Inventor: SEO, Jae Hong, Gwacheon-si Gyeonggi-do 13803 (KR); NAM, Kee Dal, Seoul 02467 (KR); KIM, Ji Young, Seoul 08311 (KR); KIM, Yoon Jae, Paju-si Gyeonggi-do 10907 (KR); PARK, Min Su, Seoul 08298 (KR); KANG, Yong Koo, Seoul 04426 (KR)
(74) Representative: ZBM Patents - Zea, Barlocci & Markvardsen
(86) International application number: PCT/KR2021/014550
(87) International publication number: WO 2022/086110

(57) **Abstract**

The present invention relates to a thiobenzimidazole derivative or a pharmaceutically acceptable salt thereof, and a composition for preventing or treating cancer, comprising the derivative as an active ingredient. The thiobenzimidazole derivative of the present invention exhibits cell toxicity by blocking the cell cycle of a cancer cell and inducing apoptosis when administered to an individual, as the derivative inhibits tubulin polymerization by being activated in the cancer cell, and, thus, the derivative can be used for prevention or treatment of cancer, desirably for prevention or treatment of triple-negative breast cancer.

## Description

### Technical Field

The present disclosure relates to a thiobenzimidazole derivative or a pharmaceutically acceptable salt thereof, and uses thereof.

### Background Art

Triple-negative breast cancer (TNBC; ER-, PR-, HER2-) patients account for 10% to 15% of all breast cancer patients, and lack hormone receptors (estrogen receptor (ER) and progesterone receptor (PR)) and HER2 proteins, so as not to benefit from hormone therapy or HER2-targeted therapies. Currently, the standard therapy for triple-negative breast cancer is completely dependent on general cytotoxic anticancer agents (Taxene-based or Anthracycline-based). Since there are no established targeted therapies, treatment strategies for other subtypes are less diverse than for breast cancer. It is more serious that after surgery or chemotherapy, recurrence occurs within 2 to 3 years in most patients, and metastasis to other organs such as lung, liver, brain, and bone is easily induced, which affects the reduction of the survival rate of patients.

The 5-years overall survival rate of patients diagnosed with stage-III is 55% or less, and the 5-years overall survival rate of patients with advanced-stage cancer is very low as 30% or less. The triple-negative breast cancer is a very serious disease that most of these patients ultimately lead to death within a few years.

A microtubule is a major component of the cytoskeleton and consists of a tubulin heteropolymer consisting of α subunits and β subunits. Microtubules perform various cellular functions, such as intracellular transport, polarity maintenance, intracellular signal transduction, and cell migration and proliferation. During mitosis of the cell, the chromosomes are disposed in the center of the cell by forming spindle fibers and then separated into two poles. If the spindle fibers do not function properly, cell division is inhibited and apoptosis occurs, so that the microtubules are attracting attention as a target of anticancer agents.

Drugs targeting the microtubules are largely divided into two groups: drugs serving to stabilize microtubules and drugs serving to destabilize microtubules. First, a microtubule stabilizer includes taxane, paclitaxel (Taxol), and decetaxel and serves to prevent depolymerization of microtubules and enhance polymerization. Most microtubule stabilizing substances bind to a taxane-binding site or to an overlapping site of β-tubulin. Second, a microtubule destabilizer includes colchicines, and vinca alkaloids, and binds to a colchicine binding site or vinca binding site. Drugs that target these microtubules themselves are effective at a lower concentration than drugs that affect microtubule polymers, and as a result, the drugs are the same in the inhibition of cell mitosis. Therefore, it is required to develop a potential tubulin polymerization inhibitor as an anticancer agent.

Meanwhile, flubendazole and albendazole are tubulin polymerization inhibitors, which are drugs commercially available as anthelmintics, but may confirm apoptosis by cell cycle arrest, and exhibit the apoptosis effect even on slow-growing cancer cells and thus have attracted attention as therapies for various cancers. However, there is a limitation that it is difficult to be absorbed in the body due to its low solubility in water, and side effects such as an increase in liver enzyme levels are exhibited when ingested in large amounts, and thus, it is required to develop derivatives with a new structure.

Accordingly, the present inventors found that a novel thiobenzimidazole derivative or a pharmaceutically acceptable salt thereof was a tubulin polymerization inhibitor that arrested the cell cycle of cancer cells (cell cycle arrest) to induce apoptosis, and then completed the present disclosure.

### Disclosure of the Invention

### Technical Goals

A technical aspect of the present disclosure is to provide a thiobenzimidazole derivative or a pharmaceutically acceptable salt thereof.

A technical aspect of the present disclosure is to provide a method for preparing the thiobenzimidazole derivative or the pharmaceutically acceptable salt thereof.

Another aspect of the present disclosure is to provide a pharmaceutical composition for preventing or treating cancer including the thiobenzimidazole derivative or the pharmaceutically acceptable salt thereof as an active ingredient.

However, technical goals to be achieved are not limited to those described above, and other goals not mentioned above are clearly understood by one of ordinary skill in the art from the following description.

### Technical Solutions

According to one embodiment of the present disclosure, the present disclosure provides a thiobenzimidazole derivative represented by Chemical Formula 1 below or a racemate, isomer, solvate or pharmaceutically acceptable salt thereof: 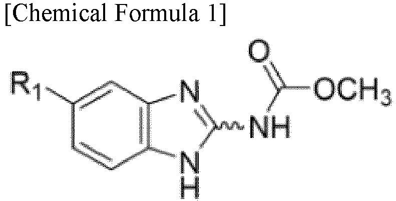 in Chemical Formula 1,
R₁ is -S-R₂ or -S-S-R₂, and
R₂ may be any one selected from the group consisting of substituted or unsubstituted cyclic alkyl, heterocycloalkyl, aryl, heteroaryl, and alkylaryl groups, desirably a substituted or unsubstituted C₃ to C₂₀ aryl group, a substituted or unsubstituted C₃ to C₂₀ heteroaryl group, or a substituted or unsubstituted C₃ to C₂₀ alkylaryl group.

According to one embodiment of the present disclosure, in Chemical Formula 1 above, the substituted aryl group, heteroaryl group, or alkylaryl group may be substituted with at least one selected from the group consisting of substituted or unsubstituted alkyl, cyclic alkyl, heterocycloalkyl, aryl group, heteroaryl, halogen, hydroxy, alkoxy, and amino groups, desirably a substituted or unsubstituted C₃ to C₁₂ heterocycloalkyl group, a substituted or unsubstituted C₃ to C₁₂ heteroaryl group, or a halogen group.

According to another embodiment of the present disclosure, the thiobenzimidazole derivative represented by Chemical Formula 1 above may be any one selected from the group consisting of the following compounds: 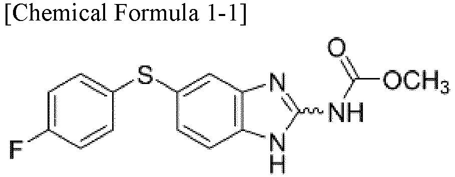 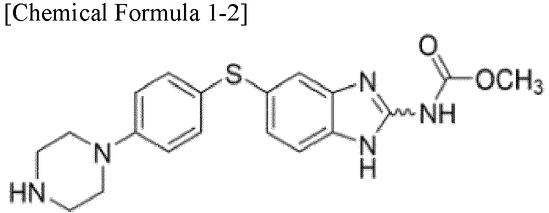 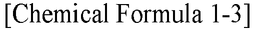 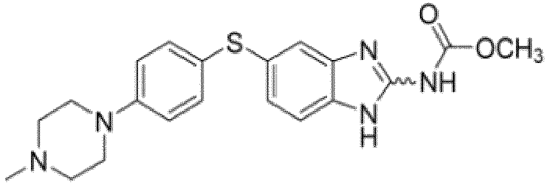 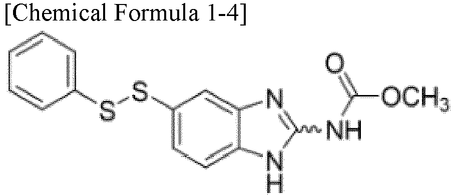 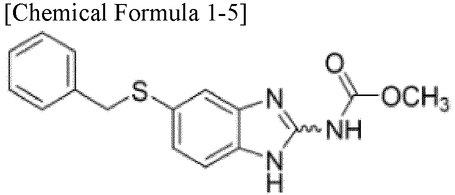 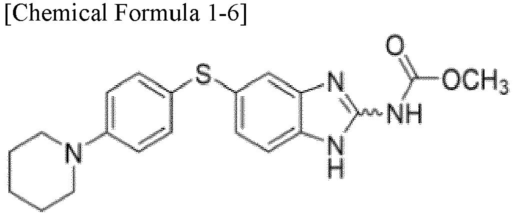 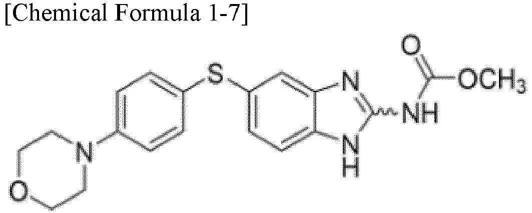 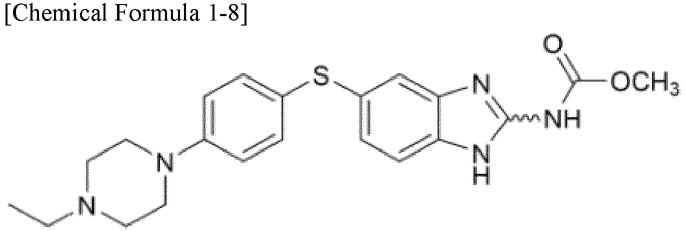 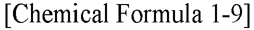 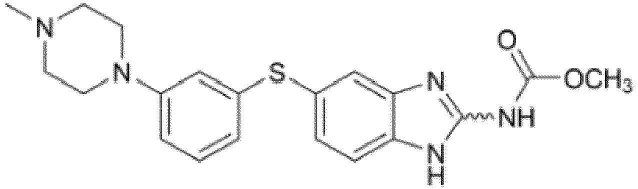 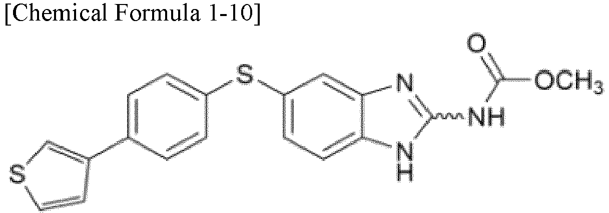 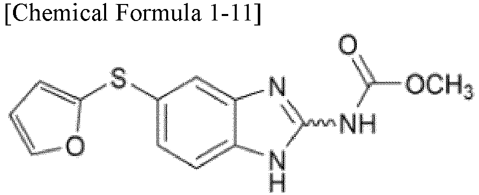 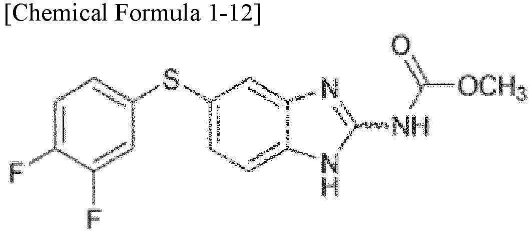 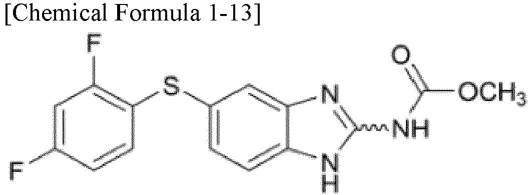 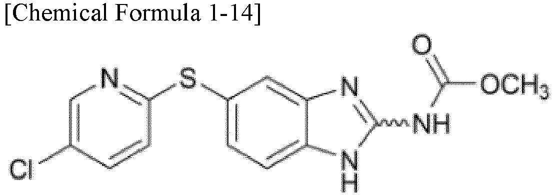 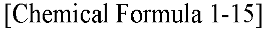 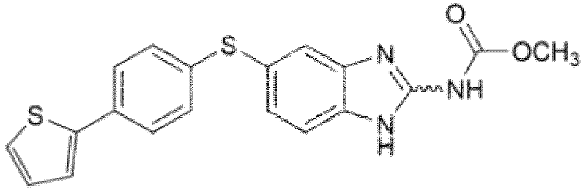 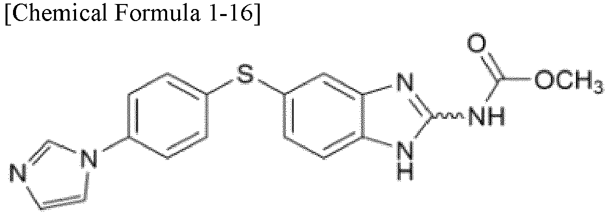 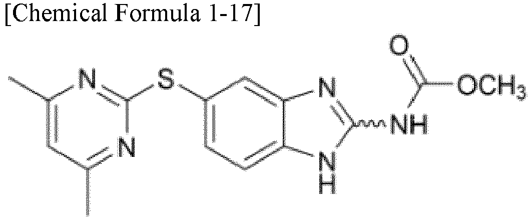 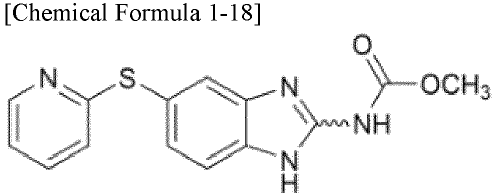 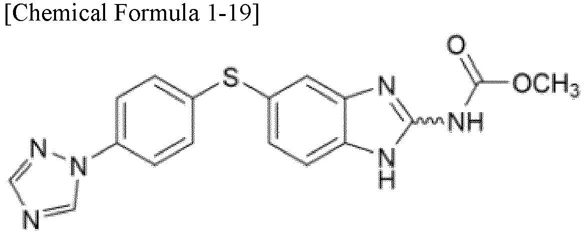 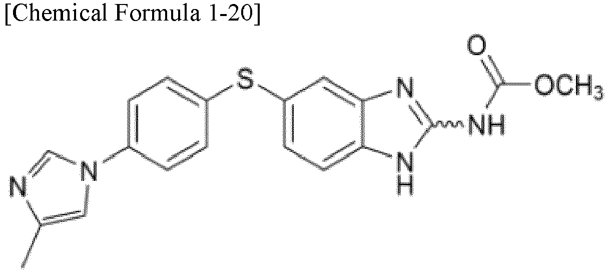 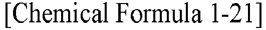 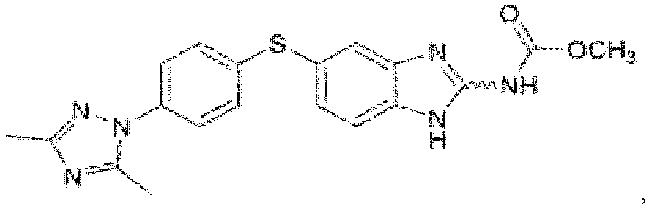 and

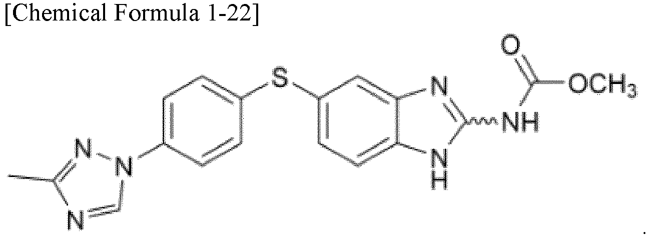

According to yet another embodiment of the present disclosure, the thiobenzimidazole derivative may inhibit tubulin polymerization.

According to still another embodiment of the present disclosure, the pharmaceutically acceptable salt of the thiobenzimidazole derivative may be at least any one selected from the group consisting of hydrochloride, bromate, sulfate, phosphate, nitrate, citrate, acetate, lactate, tartrate, maleate, gluconate, succinate, formate, trifluoroacetate, oxalate, fumarate, glutarate, adipate, methanesulfonate, benzenesulfonate, p-toluenesulfonate, camphorsulfonate, sodium salt, potassium salt, lithium salt, calcium salt, and magnesium salt, desirably hydrochloride.

Another aspect of the present disclosure provides a method for preparing hydrochloride of a thiobenzimidazole derivative including steps of:
(1) preparing a suspension of a thiobenzimidazole derivative represented by Chemical Formula 1 below; 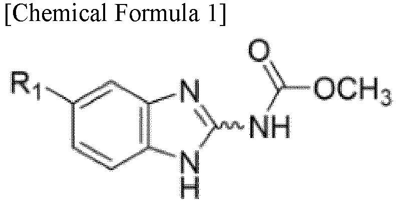
(2) injecting hydrogen chloride (HCl) into the suspension and evaporating under reduced pressure;
(3) adding isopropyl alcohol to the product of step (2) and cooling after heating; and
(4) adding, stirring, and then filtering isopropyl ether to the product of step (3).

Yet another aspect of the present disclosure provides a pharmaceutical composition for preventing or treating cancer including the thiobenzimidazole derivative or the pharmaceutically acceptable salt thereof as an active ingredient.

Still another aspect of the present disclosure provides a method for preventing or treating cancer, including administering the thiobenzimidazole derivative or the pharmaceutically acceptable salt thereof to a subject.

Still yet another aspect of the present disclosure provides a use of the thiobenzimidazole derivative or the pharmaceutically acceptable salt thereof for preparing a drug for prevention or treatment of cancer.

Still yet another aspect of the present disclosure provides a method for diagnosing cancer, including administering the thiobenzimidazole derivative or the pharmaceutically acceptable salt thereof to a subject.

Still yet another aspect of the present disclosure provides a use of the thiobenzimidazole derivative or the pharmaceutically acceptable salt thereof for preparing a drug for diagnosis of cancer.

As an embodiment of the present disclosure, the pharmaceutical composition may induce apoptosis by arresting the cell cycle of cancer cells (cell cycle arrest).

As another embodiment of the present disclosure, the cancer may be at least one selected from the group consisting of skin cancer, breast cancer, cervical cancer, esophageal cancer, stomach cancer, brain tumor, colon cancer, rectal cancer, colorectal cancer, lung cancer, ovarian cancer, cervical cancer, endometrial cancer, vulvar cancer, kidney cancer, blood cancer, pancreatic cancer, prostate cancer, testicular cancer, larynx cancer, head and neck cancer, thyroid cancer, liver cancer, bladder cancer, osteosarcoma, lymphoma, leukemia, thymus cancer, urethral cancer, and bronchial cancer, desirably breast cancer, and more desirably triple-negative breast cancer.

### Effects

The present disclosure relates to a thiobenzimidazole derivative or a pharmaceutically acceptable salt thereof, and a composition for preventing or treating cancer including the derivative as an active ingredient. The thiobenzimidazole derivative of the present disclosure is activated in cancer cells to inhibit tubulin polymerization, and exhibits cytotoxicity by blocking the cell cycle of cancer and inducing apoptosis when administered to an individual, and thus, may be used for prevention or treatment of cancer, desirably for prevention or treatment of triple-negative breast cancer.

### Brief Description of Drawings

FIG. 1 illustrates a result of cell viability after treating thiobenzimidazole derivatives of Chemical Formulas 1-1, 1-2, 1-3 and 1-5 in a triple-negative breast cancer (TNBC) cell line MDA-MB-231.
FIG. 2 illustrates a result of cell viability after treating thiobenzimidazole derivatives of Chemical Formulas 1-1, 1-2, 1-3 and 1-5 in an HER2-positive breast cancer (HER2+ BC) cell line JIMT-1.
FIG. 3 illustrates a result of cell viability after treating thiobenzimidazole derivatives of Chemical Formulas 1-6, 1-7, 1-8, 1-10, 1-11, 1-12, 1-13 and 1-14 in a triple-negative breast cancer (TNBC) cell line MDA-MB-231.
FIG. 4 illustrates a result of cell viability after treating thiobenzimidazole derivatives of Chemical Formulas 1-6, 1-7, 1-8, 1-10, 1-11, 1-12, 1-13 and 1-14 in an HER2-positive breast cancer cell line JIMT-1.
FIG. 5 illustrates a result of cell viability after treating thiobenzimidazole derivatives of Chemical Formulas 1-15, 1-16, 1-17, 1-19, 1-20, 1-21, and 1-22 in a triple-negative breast cancer (TNBC) cell line MDA-MB-231.
FIG. 6 illustrates a result of cell viability after treating thiobenzimidazole derivatives of Chemical Formulas 1-15, 1-16, 1-17, 1-19, 1-20, 1-21, and 1-22 in an HER2-positive breast cancer cell line JIMT-1.
FIG. 7 illustrates a result of cell viability after treating a thiobenzimidazole derivative of Chemical Formula 1-3 in HER2-positive breast cancer cell lines SKBR3, BT474, and JIMT-1.
FIG. 8 illustrates a result of confirming the induction of death of cancer cells (Sub-G1 accumulation) and G2/M phase cell cycle arrest by a thiobenzimidazole derivative of Chemical Formula 1-3.
FIG. 9 illustrates a result of confirming early and late apoptosis of cancer cells by a thiobenzimidazole derivative of Chemical Formula 1-3.
FIG. 10 illustrates a result of cell viability after treating a thiobenzimidazole derivative of Chemical Formula 1-3 and its hydrochloride (Compound 1-23) in HER2-positive breast cancer cell lines BT474 and JIMT-1.
FIG. 11 illustrates a result of cell viability after treating hydrochloride (Compound 1-23) of the thiobenzimidazole derivative of Chemical Formula 1-3 in triple-negative breast cancer (TNBC) cell lines MDA-MB-231, BT549 and 4T1.
FIG. 12 illustrates a result of performing western blot after treating hydrochloride (Compound 1-23) of the thiobenzimidazole derivative of Chemical Formula 1-3 in a triple-negative breast cancer (TNBC) cell line MDA-MB-231 and an HER2-positive breast cancer cell line JIMT-1.
FIG. 13 illustrates a result of cell viability after treating hydrochloride (Compound 1-23) of the thiobenzimidazole derivative of Chemical Formula 1-3 in a hematological cancer cell line HL-60, a colorectal cancer cell line HCT116, and non-small cell lung cancer cell lines H1299 and A549.
FIG. 14 illustrates a result of cell viability after treating hydrochloride (Compound 1-23) of the thiobenzimidazole derivative of Chemical Formula 1-3 in an ovarian cancer cell line SKOV3, a prostate cancer cell line Du145, and a liver cancer cell line HepG2.

### Best Mode for Carrying Out the Invention

The present inventors made intensive research on a thiobenzimidazole derivative or a pharmaceutically acceptable salt thereof, and as a result, confirmed the anticancer activity of the derivative and then completed the present disclosure.

More specifically, the present inventors confirmed that the thiobenzimidazole derivative or the pharmaceutically acceptable salt thereof was activated in cancer cells to inhibit tubulin polymerization and exhibited c ell toxicity by blocking the cell cycle of cancer and inducing apoptosis.

From the results, the present disclosure may provide a thiobenzimidazole derivative represented by Chemical Formula 1 below or a pharmaceutically acceptable salt thereof. 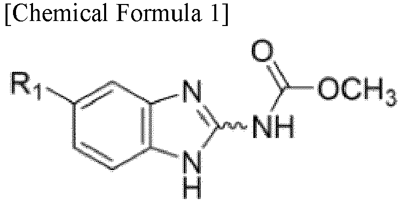 in Chemical Formula 1 above,
R₁ is -S-R₂ or -S-S-R₂, and
R₂ may be any one selected from the group consisting of a substituted or unsubstituted C₃ to C₂₀ aryl group, a substituted or unsubstituted C₃ to C₂₀ heteroaryl group, and a substituted or unsubstituted C₃ to C₂₀ alkylaryl group.

In the present disclosure, the term "substitution" refers to a reaction in which atoms or atom groups included in the molecule of the compound are replaced with other atoms or atom groups.

In the present disclosure, the term "acyclic" refers to a molecule having an acyclic structure, and the acyclic structure is a chemical structure in which carbon atoms are linked to each other in a chain shape, and has a straight chain shape and a branched shape.

In the present disclosure, the term "cyclic" refers to a structure in which both ends concatenated in the backbone of an organic compound are linked to each other to form a ring shape.

In the present disclosure, the term "acyclic or cyclic alkyl group" means a monovalent linear or branched or cyclic saturated hydrocarbon residue having 1 to 20 carbon atoms and consisting only of carbon and hydrogen atoms. Examples of such an alkyl group include methyl, ethyl, propyl, isopropyl, butyl, isobutyl, 2-butyl, 3-butyl, pentyl, n-hexyl, cyclobutyl, cyclopentyl, and cyclohexyl groups, but are not limited thereto.

In the present disclosure, the term "heterocycloalkyl group" usually refers to saturated or unsaturated (but, not aromatic) cyclohydrocarbon, which may optionally be unsubstituted, monosubstituted or polysubstituted, and in its structure, at least one is selected from heteroatoms of N, O or S.

In the present disclosure, the term "aryl group" refers to an unsaturated aromatic cyclic compound having 3 to 12 carbon atoms with a single ring (e.g., phenyl) or a plurality of condensed rings (e.g., naphthyl). Examples of such an aryl group include phenyl, and naphthyl, but are not limited thereto.

In the present disclosure, the term "heteroaryl group" refers to a single ring or a plurality of condensed rings having at least one heteroatom of N, O or S among atoms constituting the ring. Examples of such a heteroaryl group include a pyridyl group, a pyrimidinyl group, a pyrazinyl group, an oxazolyl group, and a furyl group, but are not limited thereto.

In the present disclosure, the term "alkoxy group" means an oxygen-bound alkyl group (-O-R). Examples of such an alkoxy group include a methoxy group, an ethoxy group, a propoxy group, and a butoxy group, but are not limited thereto.

In the present disclosure, the term "halogen group" may be fluorine (F), chloride (Cl), bromine (Br), iodine (I), or the like.

In the present disclosure, the R₂ may be more specifically 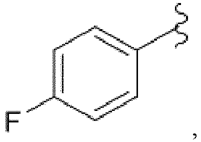 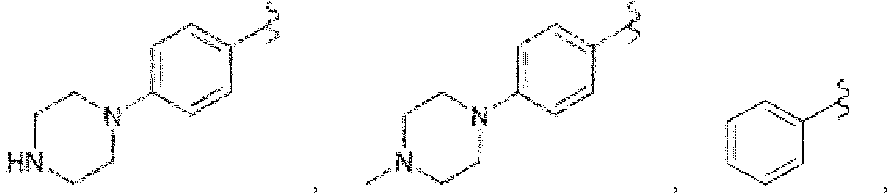 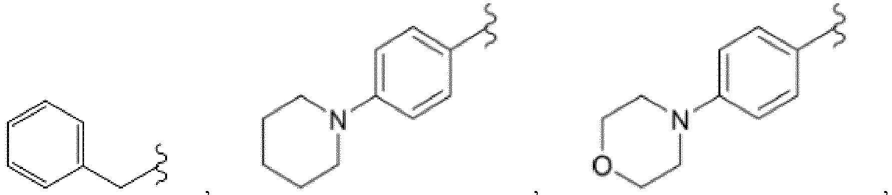 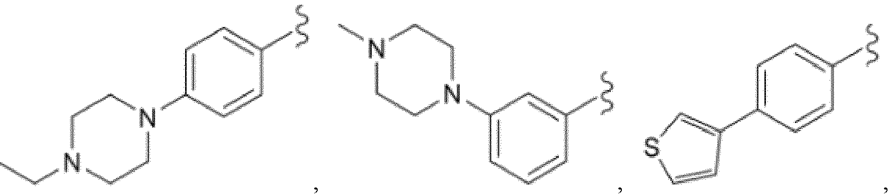 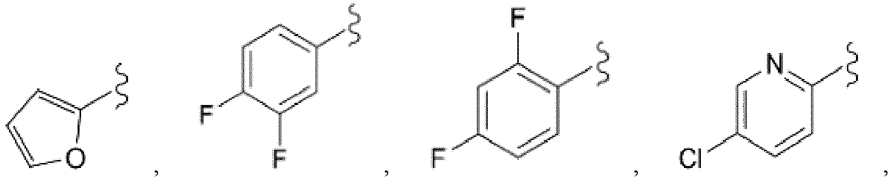 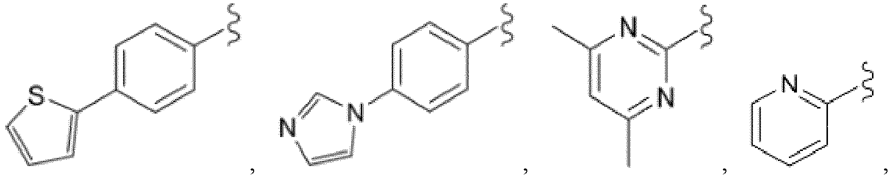 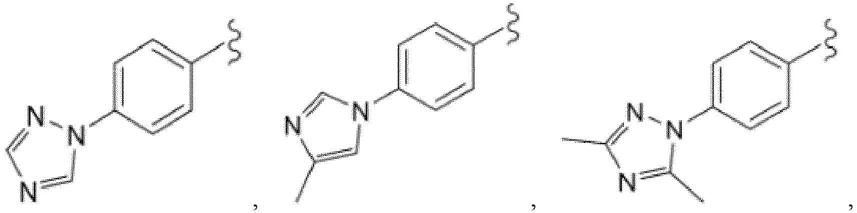 or

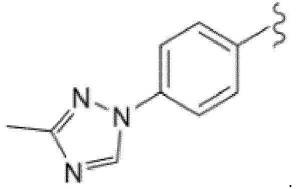

As a preferred embodiment of the present disclosure, the compound represented by Chemical Formula 1 above is desirably at least one selected from the group consisting of the following compounds: 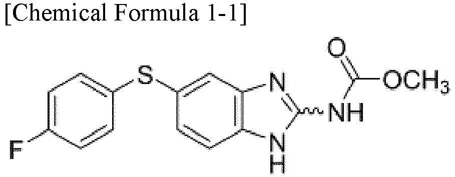 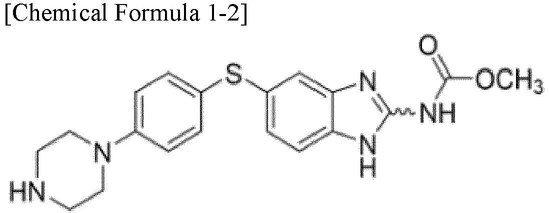 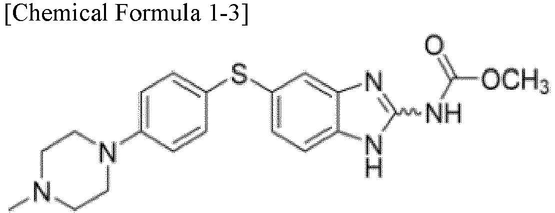 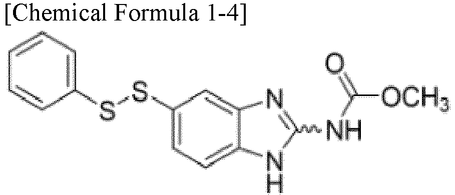 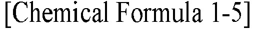 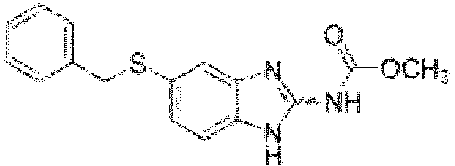 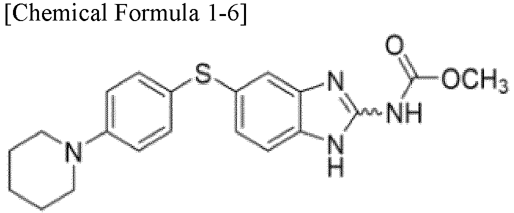 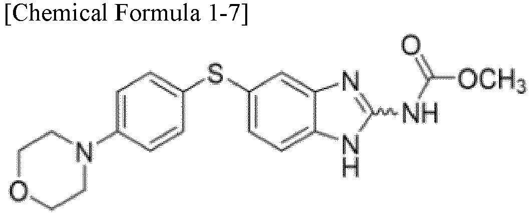 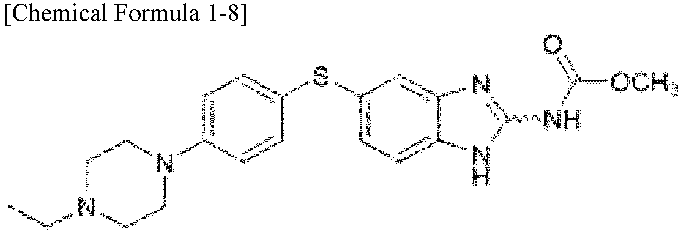 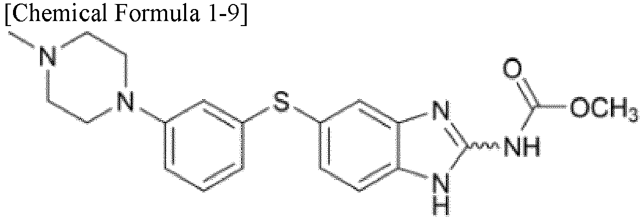 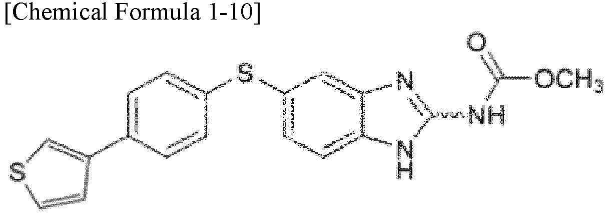 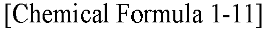 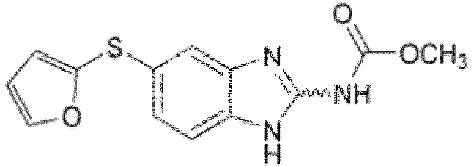 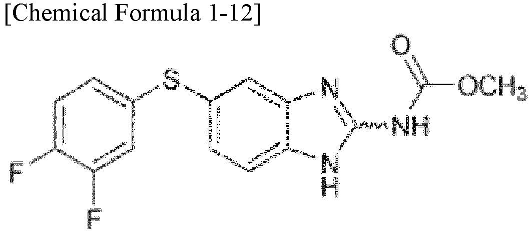 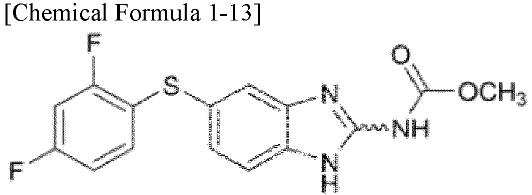 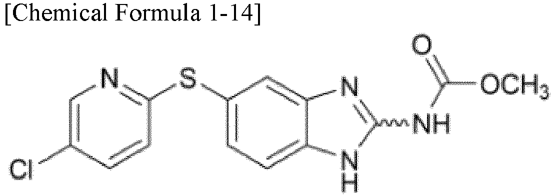 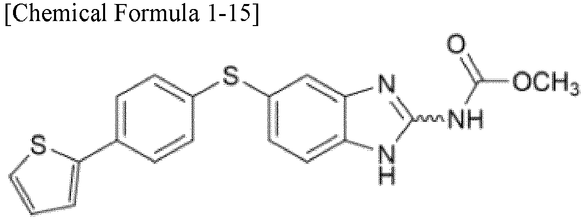 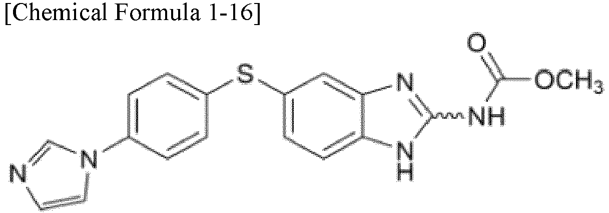 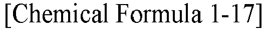 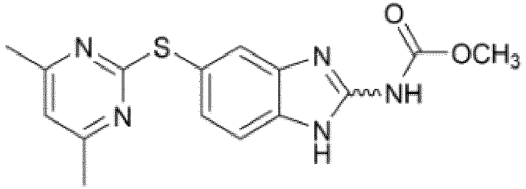 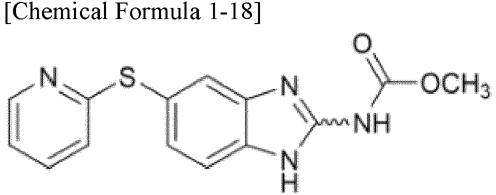 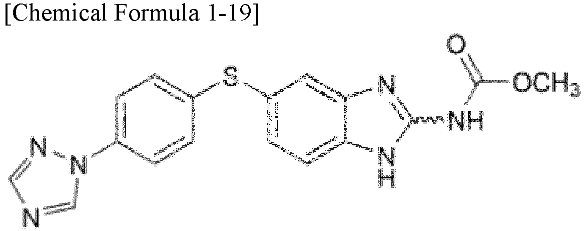 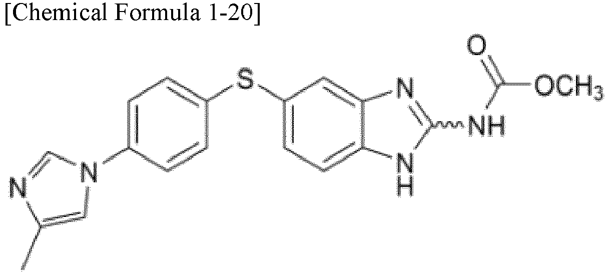 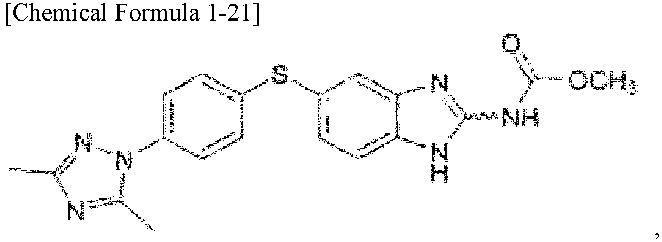 and

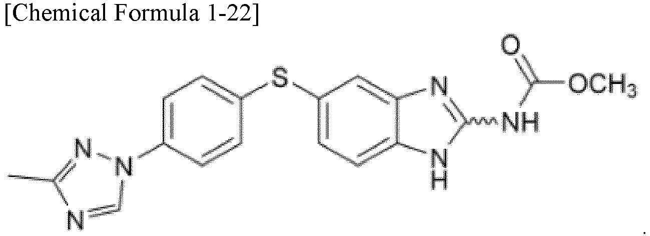

The thiobenzimidazole derivative of the present disclosure or the pharmaceutically acceptable salt thereof is specifically activated in cancer cells to inhibit tubulin polymerization and induces apoptosis, and thus, may be used as a pharmaceutical composition for preventing or treating cancer including the thiobenzimidazole derivative or the pharmaceutically acceptable salt thereof as an active ingredient.

In the present disclosure, the term "pharmaceutically acceptable salt" means a formulation of a compound that does not cause serious irritation to an organism to which the compound is administered and does not impair the biological activity and physical properties of the compound. The pharmaceutically acceptable salt may be obtained by reacting the compound of the present disclosure with inorganic acid such as hydrochloric acid, hydrobromic acid, sulfuric acid, nitric acid, and phosphoric acid; sulfonic acid such as methanesulfonic acid, ethanesulfonic acid, and p-toluenesulfonic acid; and organic carbonic acid such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, and salicylic acid. In addition, the pharmaceutically acceptable salt may also be obtained by reacting the compound of the present disclosure with bases to form alkali metal salts such as ammonium salt, sodium or potassium salt; salts such as alkaline earth metal salts such as calcium or magnesium salt; salts of organic bases such as dicyclohexylamine, N-methyl-D-glucamine and tris(hydroxymethyl) methylamine; and amino acid salts such as arginine and lysine.

In addition, the thiobenzimidazole derivative or the pharmaceutically acceptable salt thereof may include not only pharmaceutically acceptable salts, but also all salts, hydrates and solvates that may be prepared by conventional methods.

In addition, the present disclosure may provide a method for preventing, treating, and/or diagnosing cancer including administering the thiobenzimidazole derivative or the pharmaceutically acceptable salt thereof to a subject.

In the present disclosure, the term "prevention" means all actions that inhibit or delay the occurrence, spread or recurrence of cancer by administering the composition of the present disclosure, and the "treatment" means all actions that improve or beneficially change the symptoms of the disease by administering the composition of the present disclosure.

As used herein, the term "pharmaceutical composition" means a composition prepared for preventing or treating the disease, and may be formulated and used in various forms according to conventional methods. For example, the pharmaceutical composition may be prepared for oral formulations, such as powders, granules, tablets, capsules, suspensions, emulsions, and syrups, and formulated and used in the form of external preparations, suppositories, and sterile injection solutions.

In the present disclosure, "included as the active ingredient" means that the corresponding ingredient is included in an amount required or sufficient to realize a desired biological effect. In actual application, the amount to be included as an active ingredient is determined as an amount for treating a target disease, and may be determined in consideration of factors that do not cause other toxicity. For example, the amount may vary according to various factors such as a disease or condition to be treated, a type of composition to be administered, the size of a subject, or the severity of a disease or condition. Effective amounts of individual compositions may be determined empirically without undue experimentation by those skilled in the art to which the present disclosure pertains.

In addition, the pharmaceutical composition of the present disclosure may further include one or more pharmaceutically acceptable carriers in addition to the above-described active ingredients according to each formulation.

The pharmaceutically acceptable carrier may be saline, sterile water, a Ringer's solution, buffered saline, a dextrose solution, a maltodextrin solution, glycerol, ethanol, and mixtures of at least one ingredient thereof, and if necessary, may also further include other conventional additives such as antioxidants, buffers, and bacteriostats. In addition, the pharmaceutical composition may also be prepared in injectable formulations such as aqueous solutions, suspensions, and emulsions, pills, capsules, granules, or tablets by further adding a diluent, a dispersant, a surfactant, a binder, and a lubricant. Furthermore, the pharmaceutical composition may also be desirably prepared according to each disease or ingredient by a suitable method of the art, or using a method disclosed in Remington's Pharmaceutical Science (Mack Publishing Company, Easton PA).

The composition of the present disclosure may be administered orally or parenterally in a pharmaceutically effective amount according to a desired method. The term 'pharmaceutically effective dose' used herein refers to an amount enough to treat the disease at a reasonable benefit/risk ratio applicable to medical treatment. The effective dose level may be determined according to factors including the health condition of a patient, the severity, the activity of a drug, the sensitivity to a drug, an administration method, a time of administration, a route of administration, an emission rate, duration of treatment, and simultaneously used drugs, and other factors well-known in the medical field.

Accordingly, the pharmaceutical composition of the present disclosure may be administered to the subject to prevent, treat, and/or diagnose cancer. The cancer may be skin cancer, breast cancer, cervical cancer, esophageal cancer, stomach cancer, brain tumor, colon cancer, rectal cancer, colorectal cancer, lung cancer, ovarian cancer, cervical cancer, endometrial cancer, vulvar cancer, kidney cancer, blood cancer, pancreatic cancer, prostate cancer, testicular cancer, larynx cancer, head and neck cancer, thyroid cancer, liver cancer, bladder cancer, osteosarcoma, lymphoma, blood cancer, thymus cancer, urethral cancer, or bronchial cancer, but is not limited thereto. Desirably, the cancer may be cancer characterized by having acidity higher than that of normal cells and inhibiting cell toxicity by a tubulin polymerization inhibitor, and as a non-limiting example, breast cancer, and desirably triple-negative breast cancer breast cancer.

In the present disclosure, the term "subject" is any mammal, such as livestock or humans required for prevention, treatment, and/or diagnosis of cancer, but is not limited thereto, and may be desirably humans.

The pharmaceutical composition of the present disclosure may be formulated in various forms for administration to a subject, and a representative formulation for parenteral administration is an injectable formulation, desirably an isotonic aqueous solution or suspension. The injectable formulation may be prepared according to techniques known in the art by using suitable dispersing or wetting agent and suspending agent. For example, the injectable formulation may be formulated for injection by dissolving each ingredient in saline or buffer. In addition, the formulation for oral administration may include, for example, ingestible tablets, buccal tablets, troches, capsules, elixirs, suspensions, syrups, and wafers, and these formulations may include diluents (e.g., lactose, dextrose, sucrose, mannitol, sorbitol, cellulose and/or glycine) and lubricants (e.g., silica, talc, stearic acid, and magnesium or calcium salts thereof and/or polyethylene glycol) in addition to the active ingredients. The tablets may include a binder such as magnesium aluminum silicate, starch paste, gelatin, tragacanth, methylcellulose, sodium carboxymethylcellulose and/or polyvinylpyrrolidine, and in some cases, may further include a disintegrant such as starch, agar, alginic acid or sodium salt thereof, an absorbent, a coloring agent, a flavoring agent and/or a sweetening agent. The formulations may be prepared by conventional mixing, granulating or coating methods.

In addition, the pharmaceutical composition of the present disclosure may further include adjuvants such as preservatives, hydrating agents, emulsifying accelerators, and salts or buffers for osmotic pressure control, and other therapeutically useful substances, and may be formulated according to conventional methods.

The pharmaceutical composition according to the present disclosure may be administered through various routes including oral, transdermal, subcutaneous, intravenous or intramuscular route, and the dosage of the active ingredient may be appropriately selected according to several factors such as a route of administration, the age, sex, and weight of a patient, and the severity of a patient. In addition, the composition of the present disclosure may be administered in combination with known compounds capable of increasing the desired effect.

As the route of administration of the pharmaceutical composition according to the present disclosure, the pharmaceutical composition may be administered to humans and animals orally or parenterally, such as intravenously, subcutaneously, intranasally or intraperitoneally. The oral administration also includes sublingual application. The parenteral administration includes an injection method such as subcutaneous injection, intramuscular injection and intravenous injection and a dripping method.

In the pharmaceutical composition of the present disclosure, the total effective dose of the thiobenzimidazole derivative according to the present disclosure or the pharmaceutically acceptable salt thereof may be administered to a patient in a single dose, or in a plurality of doses according to a fractionated treatment protocol administered for a long period of time. In the pharmaceutical composition of the present disclosure, the content of the active ingredient may vary depending on the severity of disease, but generally, the pharmaceutical composition may be repeatedly administered several times a day at an effective dose of 100 µg to 3,000 mg per administration based on adults. However, in the concentration of the thiobenzimidazole derivative or the pharmaceutically acceptable salt thereof, the effective dose to the patient may be determined by considering various factors such as the age, weight, health condition, and sex of the subject, the severity of disease, diet and excretion rate as well as the route of administration of the drug and the number of treatments.

In addition, the pharmaceutical composition according to the present disclosure is not particularly limited to the formulation, the administration route, and the administration method thereof, as long as the effects of the present disclosure are exhibited. The pharmaceutical composition of the present disclosure may further include a known anticancer agent in addition to the thiobenzimidazole derivative or the pharmaceutically acceptable salt thereof as an active ingredient, and may be used in combination with other known therapies for treatment of these diseases.

The terms used in the embodiments are used for the purpose of description only, and should not be construed to be limited. The singular expression includes the plural expression unless the context clearly dictates otherwise. In the present disclosure, it should be understood that term "including" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as ideal or excessively formal meanings unless otherwise defined in the present disclosure.

Hereinafter, embodiments will be described in detail with reference to the accompanying drawings. However, since various modifications may be made to the embodiments, the scope of the present disclosure is not limited or restricted by these embodiments. It should be understood that all modifications, equivalents and substitutes for the embodiments are included in the scope of the present disclosure.

In addition, in the description with reference to the accompanying drawings, like components designate like reference numerals regardless of reference numerals and a duplicated description thereof will be omitted. In describing the embodiments, a detailed description of related known technologies will be omitted if it is determined that they unnecessarily make the gist of the embodiments unclear.

### [Examples]

### Example 1. Preparation of thiobenzimidazole derivative

All chemical reagents were commercially available. ¹H NMR spectra were recorded on Bruker Avance III 400 MHz and Bruker Fourier 300 MHz, and TMS was used as an internal standard.

LCMS was photographed on quadrupole Mass Spectrometer of Agilent LC/MSD 1200 series (Column: ODS 2000 (50 × 4.6 mm, 5 µm)) operating in ES (+) or (-) ionization mode; T = 30°C, flow rate = 1.5 mL/min; detected wavelength: 214 nm.

### 1.1 Synthesis of Chemical Formula 1-1

A synthetic process of a thiobenzimidazole derivative of Chemical Formula 1-1 was shown in Reaction Formula 1 below.

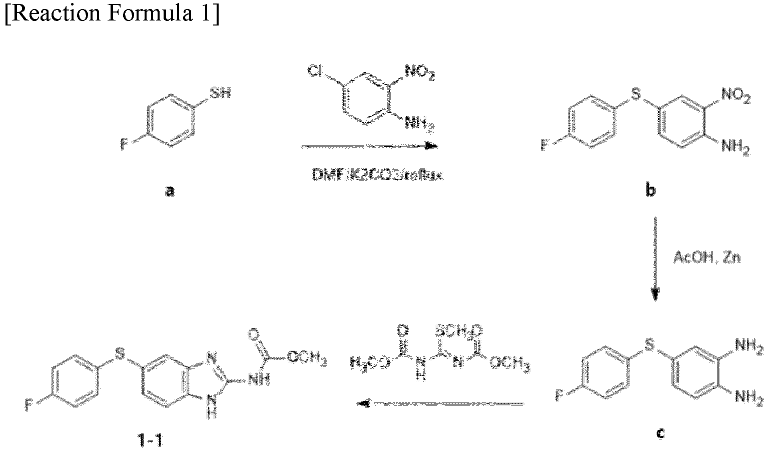

### 1.1.1. Synthesis of 5-(4-fluorophenylthio)-2-nitrobenzeneamine

4-Fluorothiol (1.1 g, 8.69 mmol) was dissolved in 10 mL, and then added with K₂CO₃ (1.24 g, 9.01 mmol). Then, 2-Nitro-5-Chloroaniline (1.5 g, 8.69 mmol) was dissolved in 3 mL and dropped. Then, the mixture was stirred at 90°C for 3 hours. After confirming the completion of the reaction by TLC, the mixture was cooled to room temperature. After pouring 50 mL of purified water, the mixture was extracted with 100 mL of ethyl acetate. The extract was dried and filtered with MgSO₄ anhydrous, and then distilled under reduced pressure to obtain a yellow solid, and the yellow solid was recrystallized with ethyl acetate/n-hexane to obtain a yellow solid of 5-(4-fluorophenylthio)-2-nitrobenzeneamine (1.87 g) (yield 82%).

R_{f}: 0.37 (EtOAc/n-Hexane, 1 : 5)

¹H NMR (400 MHz. CDCl₃ yield: 82%, δ, ppm): 6.05(brs, 2H, -NH₂), 6.33~8.02(m, 7H, ArH)

### 1.1.2. Synthesis of 4-(4-fluorophenylthio)benzene-1,2-diamine

5-(4-fluorophenylthio)-2-nitrobenzeneamine (1.0 g, 0.378 mmol) was dissolved in 20 mL of acetic acid, and slowly added with Zn (1.23 g, 18.9 mmol) under an ice-bath. Then, the mixture was stirred at room temperature for 10 hours, and then filtered after confirming the completion of the reaction by TLC. The filtrate was distilled under reduced pressure, and extracted with 100 mL of ethyl acetate after correcting the pH to 8 with a 5 M-NaOH solution. Thereafter, the extract was dried with MgSO₄ anhydrous and then filtered and distilled under reduced pressure to obtain a dark brown liquid, and the dark brown liquid was columned to obtain a brown liquid of 4-(4-fluorophenylthio)benzene-1,2-diamine (0.84 g) (yield 95.4%).

R_{f}: 0.06 (EtOAc/n-Hexane, 1 : 4)

¹H NMR (400 MHz. CDCl₃, δ, ppm): 3.41(brs, 2H, -NH₂), 3.50(brs, 2H, -NH₂), 6.68~7.28(m, 7H, ArH)

### 1.1.3. Synthesis of methyl 5-(4-fluorophenylthio)-1H-benzo[d]imidazol-2-ylcarbamate

4-(4-fluorophenylthio)benzene-1,2-diamine (0.7 g, 2.98 mmol) and 1,3-bis(methoxycarbonyl)-S-methylisothiourea (1.6 g, 7.77 mmol) were dissolved in 5%-AcOH in ethanol 10 mL and then heated and refluxed for 4 hours. After the reaction was completed with TLC, a precipitate produced by cooling to room temperature was filtered. Upon filtration, the filtrate was sufficiently washed with MeOH and dried in vacuum at 50°C to obtain a white solid of methyl 5-(4-fluorophenyl-thio)-1H-benzo[d]imidazol-2-ylcarbamate (Chemical Formula 1-1, 1.02 g) (yield 78%).

Melting Point: 232-233°C

¹H NMR (400 MHz. CDCl₃, δ, ppm): 3.85(s, 3H, -OCH₃), 6.95~7.69(m, 7H, ArH), 11.74(brs, 2H, (-NH)₂)

### 1.2 Synthesis of Chemical Formula 1-2

A synthetic process of a thiobenzimidazole derivative of Chemical Formula 1-2 was shown in Reaction Formula 2 below.

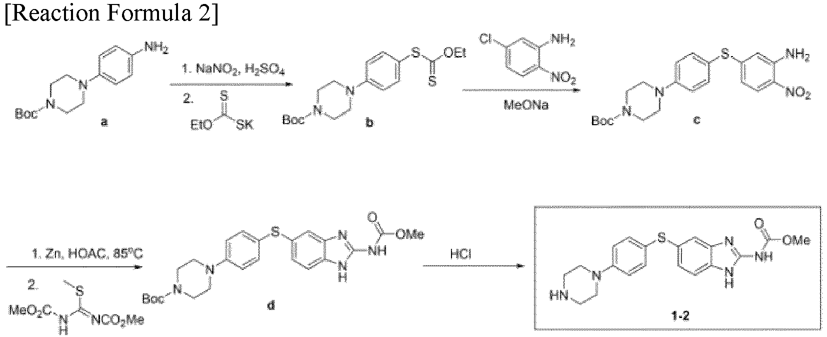

### 1.2.1. Synthesis of tert-butyl 4-(4-((ethoxycarbonothioyl)thio)phenyl)piperazine-1-carboxylate

An H₂SO₄ (1.96 g, 20.0 mmol) concentrate was added to a solution of tert-butyl 4-(4-aminophenyl)piperazine-1-carboxylate (2.77 g, 10.2 mmol) in THF/H₂O (40 mL/40 mL). Then, NaNO₂ (2.10 g, 30 mmol) dissolved in water (3.0 mL) was added slowly at - 5°C to 0°C and stirred for 2 hours. The mixture was added with potassium O-ethyl carbonodithioate (9.60 g, 60 mmol) and stirred at room temperature for 2 hours. After the reaction was completed, the mixture was poured into water (100 mL) and extracted with EA (100 mL * 3). The organic layer was dried with Na₂SO₄, concentrated and purified by reverse column (ACN/H₂O) to obtain an orange solid of 2.00 g of tert-butyl 4-(4-((ethoxycarbonothioyl)thio)phenyl)piperazine-1-carboxylate (yield 52%).

### 1.2.2. Synthesis of tert-butyl 4-(4-((3-amino-4-nitrophenyl)thio)phenyl)piperazine-1-carboxylate

A mixture of 5-chloro-2-nitroaniline (520 mg, 3.00 mmol) and NaOH (480 mg, 12 mmol) was stirred at 65°C for 16 hours in a solution of tert-butyl 4-(4-((ethoxycarbonothioyl)thio)phenyl)piperazine-1-carboxylate (1.15 g, 3.00 mmol) in THF/MeOH/H₂O (30 mL/50 mL/10 mL). After the reaction was completed, the mixture was adjusted to pH = 7-8 with AcOH, concentrated and purified by reverse column (ACN/H₂O) to obtain a brown solid of 600 mg of tert-butyl 4-(4-((3-amino-4-nitrophenyl)thio)phenyl)piperazine-1-carboxylate (yield 46%).

### 1.2.3. Synthesis of tert-butyl 4-(4-((2-((methoxycarbonyl)amino)-1H-benzo[d]imidazol-6-yl)thio)phenyl)piperazine-1-carboxylate

1,3-bis(methoxycarbonyl)-2-methyl-2-thiopseudoeura (478 mg, 2.32 mmol) and Zn powder (603 mg, 9.28 mmol) were added in a solution of tert-butyl 4-(4-((3-amino-4-nitrophenyl)thio)phenyl)piperazine-1-carboxylate (500 mg, 1.16 mmol) in AcOH (30 mL) and the mixture was stirred at 75°C for 20 hours. After the reaction was completed, the solvent was removed. The mixture was dissolved in MeOH (50 mL) and filtered, and the filtrate was concentrated and purified by reverse column (ACN/H₂O) to obtain a yellow solid of 300 mg of tert-butyl 4-(4-((2-((methoxycarbonyl)amino)-1H-benzo[d]imidazol-6-yl)thio)phenyl)piperazine-1-carboxylate (yield 53%).

### 1.2.4. Synthesis of methyl (6-((4-(piperazin-1-yl)phenyl)thio)-1H-benzo[d]imidazol-2-yl)carbamate

HCl/dioxane (6 mL, 6 mol/L) was added in a solution of tert-butyl 4-(4-((2-((methoxycarbonyl)amino)-1H-benzo[d]imidazol-6-yl)thio)phenyl)piperazine-1-carboxylate (300 mg, 0.62 mmol) in dioxane (6 mL) and the mixture was stirred at room temperature for 2 hours. After the reaction was completed, the solvent was removed and Et₃N was added. The mixture was concentrated and purified by prep-HPLC to obtain an off-white solid of 100 mg of methyl (6-((4-(piperazin-1-yl)phenyl)thio)-1H-benzo[d]imidazol-2-yl)carbamate (Chemical Formula 1-2) (yield 42%).

¹H-NMR (400 MHz, DMSO-d₆): δ 7.33 (d, 1H, J = 8.0 Hz), 7.29 (s, 1H), 7.21 (d, 1H, J = 8.8 HZ), 7.03-7.00 (m, 1H), 6.90 (d, 2H, J = 8.8 Hz), 3.74 (s, 3H), 3.06-3.03 (m, 4H), 2.82-2.79 (m, 4H)

### 1.3 Synthesis of Chemical Formula 1-3

A synthetic process of a thiobenzimidazole derivative of Chemical Formula 1-3 was shown in Reaction Formula 3 below.

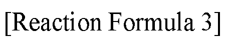

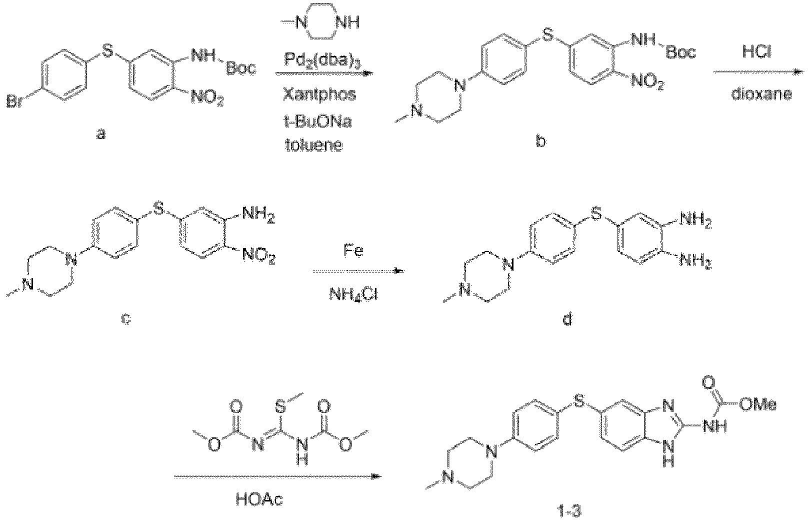

### 1.3.1. Synthesis of (5-((4-(4-methylpiperazin-1-yl)phenyl)thio)-2-nitrophenyl)carbamate

1-methylpiperazine (4.2 g, 42.4 mmol, 2.0 eq) and Pd₂(dba)₃ (1.9 g, 2.12 mmol, 0.1 eq) were added sequentially in a toluene (50 mL) solution of tert-butyl (5-((4-bromophenyl)thio)-2-nitrophenyl)carbamate (9.0 g, 21.2 mmol, 1.0 eq). Xantphos (2.0 g, 4.24 mmol, 0.2 eq) and t-BuONa (4.1 g, 42.4 mmol, 2.0 eq) were added thereto and the mixture was stirred at 100°C for 16 hours. After confirming that the reaction was completed by LCMS, the reactant was extracted with DCM, and the organic layer was concentrated and purified by TLC to synthesize a yellow solid of tert-butyl (5-((4-(4-methylpiperazin-1-yl)phenyl)thio)-2-nitrophenyl)carbamate (4 g) (yield 43%).

### 1.3.2. Synthesis of 5-((4-(4-methylpiperazin-1-yl)phenyl)thio)-2-nitroaniline

(5-((4-(4-methylpiperazin-1-yl)phenyl)thio)-2-nitrophenyl)carbamate (3 g, 6.75 mmol, 1.0 eq) was stirred in a HCl/dioxane (30 mL) solution at room temperature for 2 hours, and the completion of the reaction was confirmed by LCMS. The mixture was added with NaHCO₃, the reactant was extracted with DCM, and the organic layer was concentrated and purified by TLC to obtain a yellow solid of 5-((4-(4-methylpiperazin-1-yl)phenyl)thio)-2-nitroaniline (2.3 g) (yield 95%).

### 1.3.3. Preparation of 4-((4-(4-methylpiperazin-1-yl)phenyl)thio)benzene-1,2-diamine

Fe (1.8 g, 33.4 mmol, 5.0 eq) and NHCl (3.7 g, 66.8 mmol, 5.0 eq) were added in a EtOH (50 mL) and H₂O (10 mL) solution of 5-((4-(4-methylpiperazin-1-yl)phenyl)thio)-2-nitroaniline (2.3 g, 6.68 mmol, 1.0 eq). The mixture was stirred at 80°C for 2 hours, the completion of the reaction was confirmed by LCMS, the reactant was extracted with DCM, and then the organic layer was concentrated and purified by TLC to obtain a brown solid of 4-((4-(4-methylpiperazin-1-yl)phenyl)thio)benzene-1,2-diamine (1.9 g) (yield 90%).

¹H NMR (400 MHz, DMSO-d₆): δ 7.03 (d, J=8.8 Hz, 2H), 6.84 (d, J=8.8 Hz, 2H), 6.56 (s, 1H), 6.46 (s, 2H), 4.57-4.66 (m,4H), 3.06-3.08 (m, 4H), 2.40-2.42 (m, 4H), 2.19 (s, 1H)

### 1.3.4. Preparation of (5-((4-(4-methylpiperazin-1-yl)phenyl)thio)-1H-benzo[d]imidazol-2-yl)carbamate

1,3-bis(methoxycarbonyl)-S-methylisothiourea (575 mg, 2.79 mmol, 1.1 eq) was added in a CH₃COOH (20 mL) solution of 4-((4-(4-methylpiperazin-1-yl)phenyl)thio)benzene-1,2-diamine (800 mg, 2.54 mmol, 1.0 eq) and the mixture was stirred at 80°C for 2 hours, and the completion of the reaction was confirmed by LCMS. The reaction product mixture was dried with anhydrous Na₂SO₄ and filtered. The filtrate was concentrated and a fraction was purified by Prep-TLC to obtain a white solid of methyl (5-((4-(4-methylpiperazin-1-yl)phenyl)thio)-1H-benzo[d]imidazol-2-yl)carbamate (632 mg) (yield 63%).

¹H NMR (400 MHz, DMSO-d₆) δ 7.28-7.32 (m,2H), 7.20 (d, J=8.8 Hz, 2H), 6.99-7.01 (m, 1H), 6.91 (d, J=8.8 Hz, 2H), 3.71 (s, 3H), 3.11-3.14 (m, 4H), 2.41-2.43 (m, 4H), 2.20 (s, 3H)

### 1.4 Synthesis of Chemical Formula 1-4

A synthetic process of a thiobenzimidazole derivative of Chemical Formula 1-4 was shown in Reaction Formula 4 below.

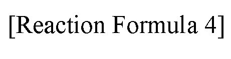

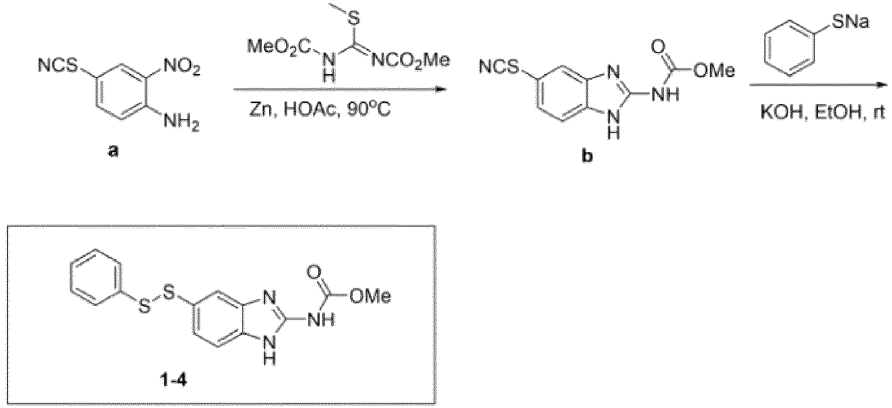

### 1.4.1. Synthesis of methyl (5-thiocyanato-1H-benzo[d]imidazol-2-yl) carbamate

Zn powder (5.00 g, 70.0 mmol) and 1,3-bis(methoxycarbonyl)-2-methyl-2-thiopseudoeura (2.70 g, 13.0 mmol) were added in a solution of 2-nitro-4-thiocyanatoaniline (1.95 g, 10.0 mmol) in AcOH (50 mL). The mixture was stirred at 90°C for 20 minutes. After the reaction was completed, the mixture was filtered. The organic layer was concentrated in vacuum. The residue was purified by reverse column (CH₃CN/H₂O) to obtain a yellow solid of 300 mg of methyl (5-thiocyanato-1H-benzo[d]imidazol-2-yl) carbamate.

### 1.4.2. Synthesis of methyl (5-(phenyldisulfanyl)-1H-benzo[d]imidazol-2-yl) carbamate

PhSNa (319 mg, 2.40 mmol) and KOH (101 mg, 1.80 mmol) were added in a solution of methyl (5-thiocyanato-1H-benzo[d]imidazol-2-yl)carbamate (300 mg, 1.20 mmol) in EtOH (120 mL). The mixture was stirred at room temperature for 16 hours. After the reaction was completed, EtOH was removed. The residue was purified by prep-HPLC to obtain an off-white solid of 90 mg of Chemical Formula 1-4 (TFA salt, yield 22%).

¹H-NMR (400 MHz, DMSO-d₆): δ 7.64 (s, 1H), 7.55-7.53 (m, 2H), 7.46-7.44 (d, 1H), 7.42-7.74 (m, 2H), 7.34-7.30 (m, 2H), 3.79 (s, 3H)

### 1.5 Synthesis of Chemical Formula 1-5

A synthetic process of a thiobenzimidazole derivative of Chemical Formula 1-5 was shown in Reaction Formula 5 below.

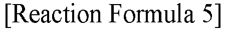

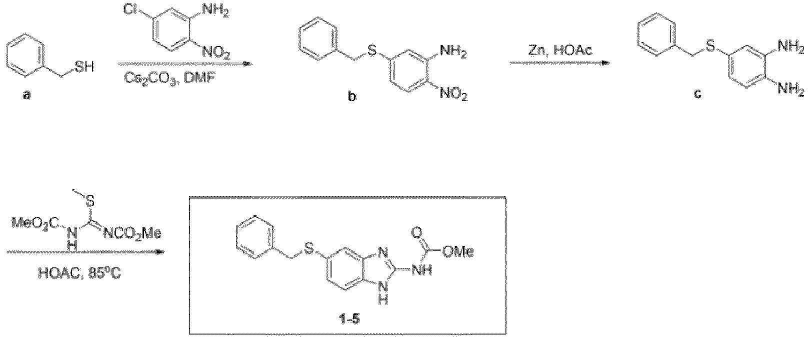

### 1.5.1. Synthesis of 5-(benzylthio)-2-nitroaniline

5-chloro-2-nitroaniline (3.40 g, 20.0 mmol) and Cs₂CO₃ (13.0 g, 40.0 mmol) were added in a solution of phenylmethanethiol (2.40 g, 20.0 mmol) dissolved in DMSO (150 mL), and the mixture was stirred at 80°C for 16 hours. After the reaction was completed, the mixture was poured into water (700 mL), extracted with EA (400 mL * 3), dried with Na₂SO₄, concentrated and purified by reverse column (ACN/H₂O) to obtain red oil of 6.00 g of 5-(benzylthio)-2-nitroaniline.

### 1.5.2. Synthesis of 4-(benzylthio)benzene-1,2-diamine

Zn (4.00 g, 61.0 mmol) was added in a solution of 5-(benzylthio)-2-nitroaniline (2.00 g, 7.70 mmol) in AcOH (50 mL). The mixture was stirred at 90°C for 3 hours. After the reaction was completed, the mixture was filtered, and the filtrate was concentrated to obtain brown oil of 2.20 g of 4-(benzylthio)benzene-1,2-diamine.

### 1.5.3. Preparation of methyl (5-(benzylthio)-1H-benzo[d]imidazol-2-yl) carbamate

1,3-bis(methoxycarbonyl)-2-methyl-2-thiopseudoeura (1.50 g, 7.20 mmol) was added in a solution of 4-(benzylthio)benzene-1,2-diamine (2.00 g, 8.70 mmol) in AcOH (40 mL). The mixture was stirred at 85°C for 16 hours. After the reaction was completed, AcOH was removed and the mixture was extracted with EA (50 mL * 3). The organic layer was dried with Na₂SO₄ and concentrated. The crude was purified by prep-HPLC to obtain a yellow solid of 75 mg of methyl (5-(benzylthio)-1H-benzo[d]imidazol-2-yl) carbamate (Chemical Formula 1-5) (yield 11%).

¹H-NMR (400 MHz, DMSO-d₆): δ11.61 (s, 1H), 7.38 (s, 1H), 7.32-7.30 (m, 1H), 7.26-7.25 (m, 1H), 7.23-7.19 (m, 4H), 7.10-7.07 (m, 1H), 4.14 (s, 2H), 3.75 (s, 3H)

### 1.6 Synthesis of Chemical Formula 1-6

A synthetic process of a thiobenzimidazole derivative of Chemical Formula 1-6 was shown in Reaction Formula 6 below.

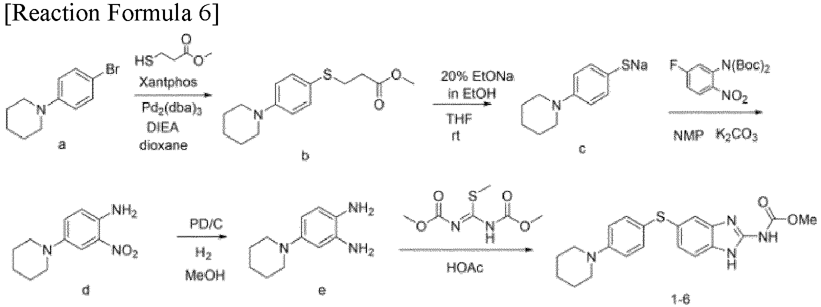

### 1.6.1. Synthesis of Methyl 3-((4-(piperidin-1-yl)phenyl)thio)propanoate

Methyl 3-mercaptopropanoate (16.13 mL, 145.81 mmol, 7.0 eq) and Xantphos (2.4 g, 4.17 mmol, 0.2 eq) were added in a dry dioxane (50 ml) solution of Compound 1-(4-bromophenyl)piperidine (5.0 g, 20.83 mmol, 1.0 eq). The mixture was continuously added with N,N-diisopropylethylamine (DIEA, 10.9 mL, 62.49 mmol, 3.0 eq) and Pd₂(dba)₃ (1.9 g, 2.08 mmol, 0.1 eq), and the reactant was stirred at 110°C for 16 hours under nitrogen gas. After the reaction was completed, the mixture was cooled to room temperature. The reactant was filtered and the filtrate was concentrated. The residue was purified by silica gel column chromatography (HE : EtOAC = 40 : 1) to obtain Compound methyl 3-((4-(piperidin-1-yl)phenyl)thio)propanoate in the form of yellow oil (5.9 g, yield 100%).

### 1.6.2. Synthesis of Sodium 4-(piperidin-1-yl)benzenethiolate

20% NaOEt (6.59 g, 19.355 mmol, 1.5 eq) was added at room temperature in a dry THF (60 mL) solution of Compound methyl 3-((4-(piperidin-1-yl)phenyl)thio)propanoate (3.6 g, 12.9 mmol, 1.0 eq). The reactant was heated to room temperature for 1 hour. After the reaction was completed, the mixture was concentrated to obtain a brown compound (3.45 g, crude material) of sodium 4-(piperidin-1-yl)benzenethiolate.

### 1.6.3. Synthesis of 2-Nitro-5-((4-(piperidin-1-yl)phenyl)thio)aniline

Compound sodium 4-(piperidin-1-yl)benzenethiolate (3.45 g, 16.05 mmol, 1.0 eq), 4-fluoro-2-di(tert-butoxycarbonyl) nitroaniline (5.98 g, 16.05 mmol, 1.0 eq) and K₂CO₃ (6.65 g, 48.15 mmol, 3.0 eq) were stirred in a solution of anhydrous 1-methyl-2-pyrrolidinone (NMP, 60 mL) at 130°C for 16 hours under nitrogen filling. After the reaction was completed, the residue was purified by silica gel column chromatography (HE : EtOAC = 20 : 1) to obtain a yellow solid of 2-nitro-5-((4-(piperidin-1-yl)phenyl)thio)aniline (630 mg).

### 1.6.4. Synthesis of 4-((4-(Piperidin-1-yl)phenyl)thio)benzene-1,2-diamine

MeOH/NH₃ (10 mL) was added in an MeOH (30 mL) solution of 2-nitro-5-((4-(piperidin-1-yl)phenyl)thio)aniline (1-4, 450 mg, 1.37 mmol, 1.0 eq) and Pd/C (110 mg) and stirred in a hydrogen (H₂) stream under atmospheric pressure at room temperature for 18 hours. The reactant was filtered and the filtrate was concentrated to obtain a purple solid (370 mg) of Compound 4-((4-(piperidin-1-yl)phenyl)thio)benzene-1,2-diamine (yield 90.5%).

¹H NMR (400 MHz, CDCl₃): δ ppm 7.21-7.25 (m, 2H), 6.85 (d, J = 6.8 Hz, 2H), 6.69-6.74 (m, 2H), 6.62 (d, J = 8.0 Hz, 1H), 3.34-3.43 (m, 4H), 3.14-3.16 (m, 4H), 1.55-1.69 (m, 6H)

1.6.5. Synthesis of (5-((4-(Piperidin-1-yl)phenyl)thio)-1H-benzo[d]imidazol-2-yl)carbamate

1,3-Bis(methoxycarbonyl)-S-methylisothiourea (76 mg, 0.367 mmol, 1.1 eq) was added in an acetic acid (5 mL) solution of Compound 4-((4-(piperidin-1-yl)phenyl)thio)benzene-1,2-diamine (100 mg, 0.334 mmol, 1.0 eq) and the mixture was stirred and reacted at 80°C for 2 hours. The reactant was extracted with DCM and washed with saturated NaHCO₃. The organic layer was dried with anhydrous Na₂SO₄ and filtered. The filtrate was concentrated and the obtained mixed product was purified by Prep-TLC to obtain a white solid product of methyl (5-((4-(piperidin-1-yl)phenyl)thio)-1H-benzo[d]imidazol-2-yl)carbamate (Chemical Formula 1-6, 20 mg) (yield 15.7%).

¹H NMR (400 MHz, DMSO-d6): δ 7.33(d, J = 8.4 Hz, 1H), 7.28 (d, J = 1.2 Hz, 1H), 7.20 (d, J = 8.8 Hz, 2H), 7.01-7.03 (m, 1H), 6.91 (d, J = 8.8 Hz, 2H), 6.08 (brs, 2H), 3.74 (s, 3H), 3.11-3.19 (m, 4H), 1.53-1.59 (m, 6H)

### 1.7 Synthesis of Chemical Formula 1-7

A synthetic process of a thiobenzimidazole derivative of Chemical Formula 1-7 was shown in Reaction Formula 7 below.

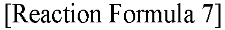

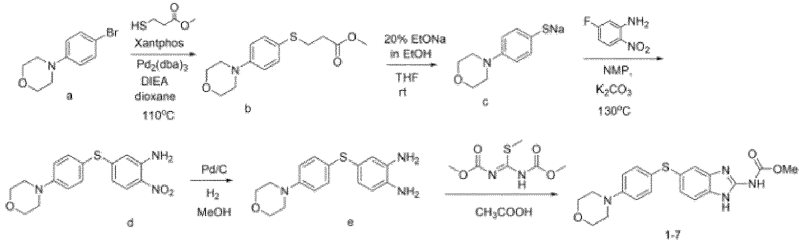

### 1.7.1. Synthesis of methyl 3-((4-morpholinophenyl)thio)propanoate

Methyl 3-mercaptopropanoate (18.6 g, 154.9 mmol, 5.0 eq) and Xantphos (3.6 g, 6.2 mmol, 0.2 eq) were added in a dry dioxane (50 mL) solution of 4-(4-bromophenyl)morpholine (7.0 g, 30.9 mmol, 1.0 eq). DIEA (12.2 g, 92.9 mmol, 3.0 eq) and Pd₂(dba)₃ (1.4 g, 1.6 mmol, 0.05 eq) were sequentially added thereto, and the reactant was reacted at 110°C for 16 hours while stirring. After the reaction was completed, the mixture was cooled to room temperature. The reactant was filtered and the filtrate was concentrated. The residue was separated and purified by silica gel column chromatography (HE : EtOAC = 8 : 1) to obtain methyl 3-((4-morpholinophenyl)thio)propanoate (7.5 g) in the form of yellow oil (yield 91.2%).

### 1.7.2. Synthesis of sodium 4-morpholinobenzenethiolate

20% NaOEt (10.7 g, 31.6 mmol, 1.5 eq) was added in a dry THF (100 mL) solution of methyl 3-((4-morpholinophenyl)thio)propanoate (7.4 g, 26.3 mmol, 1.0 eq) at room temperature. The reactant was reacted at room temperature for 1 hour. After the reaction was completed, the mixture was concentrated to obtain a brown oily liquid compound of sodium 4-morpholinobenzenethiolate (5.2 g) (yield 88.9%).

### 1.7.3. Synthesis of 4-morpholino-2-nitroaniline

5-fluoro-2-nitroaniline (4.5 g, 28.7 mmol, 1.2 eq) and K₂CO₃ (9.9 g, 71.8 mmol, 3.0 eq) were added in an anhydrous 1-methyl-2-pyrrolidinone (60 mL) solution of 4-morpholinobenzenethiolate (5.2 g, 23.9 mmol, 1.0 eq) under nitrogen filling and then stirred at 130°C for 16 hours. After the reaction was completed, the residue was purified with silica gel (HE : EtOAC = 20 : 1) by column chromatography to obtain a yellow solid (1.2 g) of Compound 4-morpholino-2-nitroaniline (yield 15.2%).

### 1.7.4. Synthesis of 4-morpholinobenzene-1,2-diamine

Pd/C (300 mg) was added to a MeOH (20 mL) solution of 4-morpholino-2-nitroaniline (1.2 g, 3.6 mmol, 1.0 eq), and stirred at room temperature in the presence of hydrogen gas (H₂) for 18 hours. The reactant was filtered and the filtrate was obtained as a purple solid of 4-morpholinobenzene-1,2-diamine (640 mg) (yield 60.1%).

¹H NMR (400 MHz, DMSO-d6): δ 7.85 (d, J = 9.2 Hz, 1H), 7.39-7.44 (m, 3H), 7.06 (d, J = 8.8 Hz, 2H), 6.52 (d, J = 1.6 Hz, 1H), 6.24-6.27 (m, 1H), 3.69 - 3.76 (m, 4H), 3.19-3.23 (m, 4H)

### 1.7.5. Synthesis of methyl (5-((4-morpholinophenyl)thio)-1H-benzo[d]imidazol-2-yl)carbamate

1,3-bis(methoxycarbonyl)-S-methylisothiourea (75 mg, 0.365 mmol, 1.1 eq) was added to an acetic acid (5 mL) solution of 4-morpholinobenzene-1,2-diamine (100 mg, 0.332 mmol, 1.0 eq) and the mixture was stirred at 80°C for 2 hours, and the completion of the reaction was confirmed by LCMS. The reactant was extracted with DCM and washed with saturated bicarbonate water, and the organic layer was dried with anhydrous Na₂SO₄ and filtered. The filtrate was concentrated and this material was purified by Prep-TLC to obtain a product as a white solid (Chemical Formula 1-7, 30 mg) (yield 23.6%).

¹H NMR (400 MHz, DMSO-d6): δ ppm 7.34 (d, J = 8.4 Hz, 1H), 7.30 (s, 1H), 7.23 (d, J = 8.8 Hz, 2H), 7.02-7.05 (m, 1H), 6.93 (d, J = 8.8 Hz, 2H), 3.74 (s, 3H), 3.71-3.73 (m, 4H), 3.10-3.12 (m, 4H)

### 1.8 Synthesis of Chemical Formula 1-8

A synthetic process of a thiobenzimidazole derivative of Chemical Formula 1-8 was shown in Reaction Formula 8 below.

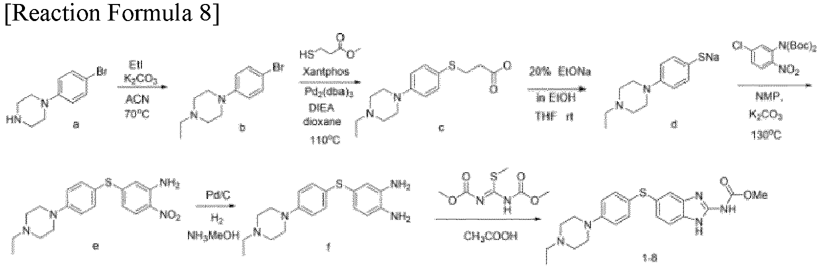

### 1.8.1. Synthesis of 1-(4-bromophenyl)-4-ethylpiperazine

Ethyl iodide (4.0 mL, 49.78 mmol, 1.2 eq) and K₂CO₃ (11.47 g, 82.96 mmol, 2.0 eq) were added to an anhydrous acetonitrile (50 mL) solution of 1-(4-bromophenyl)piperazine (10.0 g, 41.83 mmol, 1.0 eq). After the addition was completed, the reactant was stirred at 70°C for 2 hours under N₂. The reactant was filtered and the filtrate was concentrated. The residue was purified by silica gel (HE : EtOAC = 5 : 1) column chromatography to obtain a white solid (9.84 g) of Compound 1-(4-bromophenyl)-4-ethylpiperazine (yield 84.2%).

### 1.8.2. Synthesis of methyl 3-((4-(4-ethylpiperazin-1-yl)phenyl)thio)propanoate

Methyl 3-mercaptopropanoate (27.1 mL, 244.4 mmol, 7.0 eq) and Xantphos (4.04 g, 6.98 mmol, 0.2 eq) were added in a dry dioxane (50 mL) solution of 1-(4-bromophenyl)-4-ethylpiperazine (9.4 g, 34.9 mmol, 1.0 eq). DIEA (18.3 mL, 104.7 mmol, 3.0 eq) and Pd₂(dba)₃ (3.2 g, 3.5 mmol, 0.1 eq) were added thereto and then the reactant was stirred at 110°C for 16 hours under an N₂ atmosphere. The reactant was filtered and the filtrate was concentrated. The residue was purified by silica gel column chromatography (Hexane : EtOAC = 2 : 1) to obtain Compound methyl 3-((4-(4-ethylpiperazin-1-yl)phenyl)thio)propanoate (9.5 g) in the form of yellow oil (yield 88.29%).

### 1.8.3. Synthesis of sodium 4-(4-ethylpiperazin-1-yl)benzenethiolate

20% sodium ethoxide (4.64 g, 13.65 mmol, 1.5 eq) was added to a dry THF (60 mL) solution of 3-((4-(4-ethylpiperazin-1-yl)phenyl)thio)propanoate (2.81 g, 9.1 mmol, 1.0 eq) and then the reactant was heated to room temperature for 20 minutes. After the reaction was completed, the mixture was concentrated to obtain a brown solid compound of sodium 4-(4-ethylpiperazin-1-yl)benzenethiolate (2.8 g).

### 1.8.4. Synthesis of 5-((4-(4-ethylpiperazin-1-yl)phenyl)thio)-2-nitroaniline

Sodium 4-(4-ethylpiperazin-1-yl)benzenethiolate (2.8 g, 11.5 mmol, 1.0 eq) was dissolved in dry N-methylpyrrolidinedimethyl (60 mL), and sequentially added with tert-butyl (tert-butoxycarbonyl)(5-chloro-2-nitrophenyl)carbamate (4.29 g, 11.5 mmol, 1.0 eq) and K₂CO₃ (4.77 g, 34.5 mmol, 3.0 eq), and then the solution was stirred at 130°C for 16 hours under a N₂ atmosphere. After the reaction was completed, the residue was purified by silica gel column chromatography (DCM : MeOH = 20 : 1) to obtain an orange solid of 5-((4-(4-ethylpiperazin-1-yl)phenyl)thio)-2-nitroaniline (520 mg).

### 1.8.5. Synthesis of 4-((4-(4-ethylpiperazin-1-yl)phenyl)thio)benzene-1,2-diamine

Pd/C (150 mg) was added in a MeOH (15 mL) and MeOH/NH₃ (5 mL) solution of 5-((4-(4-ethylpiperazin-1-yl)phenyl)thio)-2-nitroaniline (520 mg, 1.45 mmol, 1.0 eq) and the solution was stirred at room temperature in the presence of hydrogen (H₂) for 18 hours. The reactant was filtered and the filtrate was concentrated to obtain a purple solid compound of 4-((4-(4-ethylpiperazin-1-yl)phenyl)thio)benzene-1,2-diamine (470 mg) (yield 99.5%).

¹H NMR (400 MHz, CDCl₃): δ 7.22 (d, J = 8.4 Hz, 2H), 6.82 (d, J = 8.8 Hz, 2H), 6.73-6.78 (m, 2H), 6.64 (d, J = 7.6 Hz, 1H), 3.26-3.46 (m, 8H), 2.78-2.92 (m, 4H), 1.51-1.64 (m, 2H), 1.29-1.30 (m, 3H)

### 1.8.6. Synthesis of Methyl (5-((4-(4-ethylpiperazin-1-yl)phenyl)thio)-1H-benzo[d]imidazol-2-yl)carbamate

4-((4-(4-ethylpiperazin-1-yl)phenyl)thio)benzene-1,2-diamine (100 mg, 0.305 mmol, 1.0 eq) was added in an acetic acid (5 mL) solution. The mixture of 1,3-bis(methoxycarbonyl)-S-methylisothiourea (69 mg, 0.335 mmol, 1.1 eq) was stirred at 80°C for 2 hours, and the completion of the reaction was confirmed by LCMS. The reactant was extracted with DCM and washed with saturated NaHCO₃. The filtrate was concentrated and the product was purified by Prep-TLC to obtain a white solid of methyl (5-((4-(4-ethylpiperazin-1-yl)phenyl)thio)-1H-benzo[d]imidazol-2-yl)carbamate (Chemical Formula 1-8, 44 mg) (yield 32%).

¹H NMR (400 MHz, DMSO-d₆) δ 7.33 (d, J = 8.0 Hz, 1H), 7.29 (s, 1H), 7.21 (d, J = 8.8 Hz, 2H), 7.00-7.03 (m, 1H), 6.92 (d, J = 8.8 Hz, 2H), 3.74 (s, 3H), 3.13-3.15 (m, 4H), 2.46-2.48 (m, 4H), 2.32-2.37 (m, 2H), 1.01-1.04 (m, 3H)

### 1.9 Synthesis of Chemical Formula 1-9

A synthetic process of a thiobenzimidazole derivative of Chemical Formula 1-9 was shown in Reaction Formula 9 below.

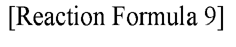

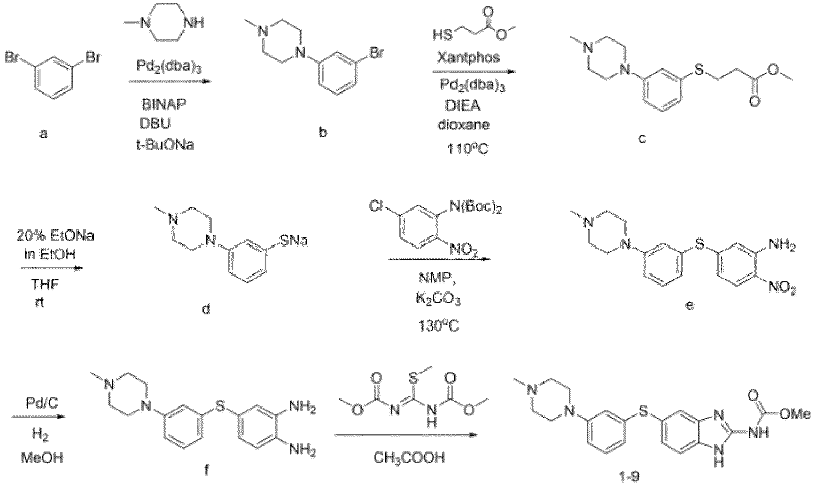

### 1.9.1. Synthesis of 1-(3-bromophenyl)-4-methylpiperazine

1-methylpiperazine (19.2 g, 192.4 mmol, 5.0 eq), BINAP (4.8 g, 7.7 mmol, 0.2 eq), DBU (17.5 g, 115.5 mmol, 3.0 eq) and Pd₂(dba)₃ (2.2 g, 1.9 mmol, 0.05 eq) were added in a toluene (100 mL) solution of 1,3-dibromobenzene (9.0 g, 38.5 mmol, 1.0 eq), respectively, and the reaction mixture was heated to 60°C, and added with freshly pulverized sodium tert-butoxide (12.9 g, 192.4 mmol, 3.0 eq) at once. The reactant was stirred at 60°C under N₂ for 16 hours and then concentrated. The residue was separated and purified by silica gel column chromatography (HE : EtOAC = 60 : 1) to obtain 1-(3-bromophenyl)-4-methylpiperazine (5.5 g) in the form of yellow oil (yield 56.3%).

### 1.9.2. Synthesis of methyl 3-((3-(4-methylpiperazin-1-yl)phenyl)thio)propanoate

Methyl 3-mercaptopropanoate (18.2 mg, 155.55 mmol, 7.0 eq) was added to an anhydrous dioxane (50 mL) solution of 1-(3-bromophenyl)-4-methylpiperazine (5.5 g, 21.65 mmol, 1.0 eq). Xantphos (2.5 g, 4.33 mmol, 0.2 eq), DIEA (8.4 g, 64.95 mmol, 3.0 eq) and Pd₂(dba)₃ (2.0 g, 2.165 mmol, 0.1 eq) were added sequentially thereto, the reaction was completed, and then the reactant was stirred for 24 hours at 110°C under N₂. The reactant was filtered and the filtrate was concentrated. The residue was purified by silica gel column chromatography (DCM : MeOH = 20 : 1) to obtain orange oil (4.5 g) of methyl 3-((3-(4-methylpiperazin-1-yl)phenyl)thio)propanoate (yield 70.7%).

### 1.9.3. Synthesis of sodium 3-(4-methylpiperazin-1-yl)benzenethiolate

20% NaOEt (3.8 g, 11.73 mmol, 1.5 eq) was added in a dry THF (60 mL) solution of methyl 3-((3-(4-methylpiperazin-1-yl)phenyl)thio)propanoate (2.3 g, 7.82 mmol, 1.0 eq). The reactant at room temperature was heated for 10 minutes. After the reaction was completed, the mixture was concentrated to obtain a brown solid (2.05 g) of sodium 3-(4-methylpiperazin-1-yl)benzenethiolate.

### 1.9.4. Synthesis of 5-((3-(4-methylpiperazin-1-yl)phenyl)thio)-2-nitroaniline

Tert-butyl (tert-butoxycarbonyl)(5-chloro-2-nitrophenyl)carbamate (3.3 g, 8.91 mmol, 1.0 eq) and K₂CO₃ (3.7 g, 26.73 mmol, 3.0 eq) were sequentially added to a dry N-methylpyrrolidinedimethyl (60 mL) solution of sodium 3-(4-ethylpiperazin-1-yl)benzenethiolate (2.05 g, 8.91 mmol, 1.0 eq) and then stirred at 130°C for 16 hours under N₂. After the reaction was completed, the residue was purified by silica gel column chromatography (DCM : MeOH = 20 : 1) to obtain an orange solid compound (3.1 g) of 5-((3-(4-methylpiperazin-1-yl)phenyl)thio)-2-nitroaniline.

### 1.9.5. Synthesis of 4-((3-(4-methylpiperazin-1-yl)phenyl)thio)benzene-1,2-diamine

5-((3-(4-methylpiperazin-1-yl)phenyl)thio)-2-nitroaniline (3.1 g, 9.0 mmol, 1.0 eq) and Pd/C (1.0 g) were added to a MeOH (60 mL) and MeOH/NH₃ (20 mL) solution and stirred under H₂ for 18 hours. The reactant was filtered and the filtrate was concentrated to obtain 4-((3-(4-methylpiperazin-1-yl)phenyl)thio)benzene-1,2-diamine (600 mg) in a gray solid form.

¹H NMR (400 MHz, CDCl₃): δ 7.07-7.12 (m, 1H), 6.84-6.89 (m, 2H), 6.78-6.80 (m, 1H), 6.66-6.70 (m, 2H), 6.60-6.63 (m, 1H), 3.49 (s, 2H), 3.36 (s, 2H), 3.14-3.17 (m, 4H), 2.52-2.55 (m,4H), 2.34(d, J= 5.6 Hz,3H)

### 1.9.6. Synthesis of methyl (5-((3-(4-methylpiperazin-1-yl)phenyl)thio)-1H-benzo[d]imidazol-2-yl)carbamate

1,3-bis(methoxycarbonyl)-S-methylisothiourea (72 mg, 0.35 mmol, 1.1 eq) was added in a CH₃COOH (10 mL) solution of 4-((3-(4-Methylpiperazin-1-yl)phenyl)thio)benzene-1,2-diamine (100 mg, 0.318 mmol, 1.0 eq). The reaction mixture was stirred at 80°C for 2 hours, and the completion of the reaction was confirmed by LCMS. The reactant was extracted with DCM and the sat. NaHCO₃ organic layer was dried with anhydrous Na₂SO₄ and filtered. The filtrate was concentrated and the mixture was purified by Prep-TLC to obtain a white solid of methyl (5-((3-(4-methylpiperazin-1-yl)phenyl)thio)-1H-benzo[d]imidazol-2-yl)carbamate (Chemical Formula 1-9, 20 mg) (yield 15.87%).

¹H NMR (400 MHz, DMSO-d₆) δ 7.49 (s, 1H), 7.43 (d, J= 8.0 Hz, 1H), 7.16-7.18 (m, 1H), 7.08-7.12 (m, 1H), 6.77 (d, J= 6 Hz, 2H), 6.47 (d, J=7.6 Hz, 1H), 3.76 (s, 3H), 3.04-3.07 (m, 4H), 2.38-2.40 (m, 4H), 2.19 (s, 3H)

### 1.10 Synthesis of Chemical Formula 1-10

A synthetic process of a thiobenzimidazole derivative of Chemical Formula 1-10 was shown in Reaction Formula 10 below.

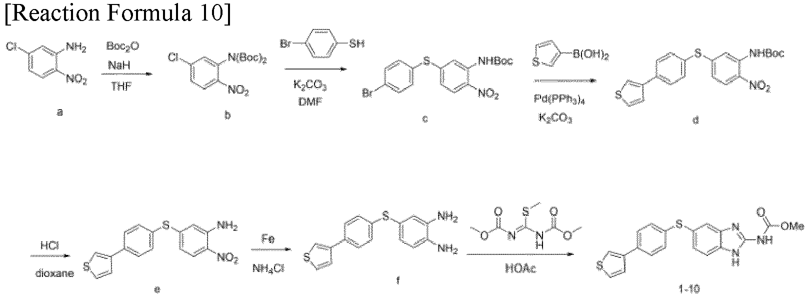

### 1.10.1. Synthesis of tert-butyl (tert-butoxycarbonyl)(5-chloro-2-nitrophenyl)carbamate

Di-tert-butyl decarbonate (50 g, 232.4 mmol, 2.0 eq) and DMAP (14 g, 116.2 mmol, 1.0 eq) were added in a THF (300 mL) solution of 5-chloro-2-nitroaniline (20 g, 116.2 mmol, 1.0 eq). The reaction mixture was stirred at 70°C for 1 hour. The completion of the reaction was confirmed by LCMS. The reactant was concentrated and purified by silica gel column chromatography (eluted at HE : EA = 10 : 1) to obtain a yellow solid of tert-butyl (tert-butoxycarbonyl)(5-chloro-2-nitrophenyl)carbamate (36 g) (yield 85%).

### 1.10.2. Synthesis of tert-butyl (5-((4-bromophenyl)thio)-2-nitrophenyl)carbamate

4-bromobenzenethiol (21 g, 112.9 mmol, 1.5 eq) and K₂CO₃ (20.7 g, 150.4 mmol, 2 eq) were added sequentially to a DMF (100 mL) solution of tert-butyl (tert-butoxycarbonyl)(5-chloro-2-nitrophenyl)carbamate (28 g, 75.2 mmol, 1.0 eq), and the reaction mixture was stirred at 100°C for 16 hours. The completion of the reaction was confirmed by LCMS. The reactant was extracted with DCM and washed with brine. The organic layer was dried and concentrated to obtain a yellow solid of tert-butyl(5-((4-bromophenyl)thio)-2-nitrophenyl)carbamate (10.2 g, yield 35%).

### 1.10.3. Synthesis of tert-butyl (2-nitro-5-((4-(thiophen-3-yl)phenyl)thio)phenyl)carbamate

Thiophen-3-yl boronic acid (2.6 g, 20.2 mmol, 2.0 eq) was added in a DMF (20 mL) and H₂O (4 mL) solution of tert-butyl (5-((4-bromophenyl)thio)-2-nitrophenyl)carbamate (4.3 g, 10.1 mmol, 1.0 eq). The mixture was sequentially added with Pd(PPh₃)₄ (1.2 g, 1.01 mmol, 0.1 eq) and K₂CO₃ (4.2 g, 30.3 mmol, 3.0 eq) and stirred at 100°C for 16 hours. The completion of the reaction was confirmed by LCMS and the reactant was extracted with DCM and washed with brine. The organic layer was dried with anhydrous Na₂SO₄ and filtered. The filtrate was concentrated, and the residue was purified by silica gel column chromatography (eluted at HE : EA = 3 : 1) to obtain a yellow solid of tert-butyl (2-nitro-5-((4-(thiophen-3-yl)phenyl)thio)phenyl)carbamate (2.7 g) (yield 62%).

### 1.10.4. Synthesis of 2-nitro-5-((4-(thiophen-3-yl)phenyl)thio)aniline

An HCl/dioxane (20 mL) solution of tert-butyl (2-nitro-5-((4-(thiophen-3-yl)phenyl)thio)phenyl)carbamate (2.6 g, 6.07 mmol, 1.0 eq) was stirred at room temperature for 2 hours. The completion of the reaction was confirmed by LCMS, and then the reaction mixture was poured into 100 mL of an ice solution of NaHCO₃. The mixture was extracted with DCM and washed with brine. The organic layer was dried with anhydrous Na₂SO₄ and filtered. The filtrate was concentrated and purified by silica gel column chromatography eluted at DCM : MeOH = 10 : 1 to obtain a yellow solid of 2-nitro-5-((4-(thiophen-3-yl)phenyl)thio)aniline (1.5 g) (yield 78%).

### 1.10.5. Synthesis of 4-((4-(thiophen-3-yl)phenyl)thio)benzene-1,2-diamine

Fe (1.1 g, 19.8 mmol, 5.0 eq) and NH₄Cl (1.1 g, 19.8 mmol, 5.0 eq) were added to a EtOH (20 mL) and H₂O (5 mL) solution of 2-nitro-5-((4-(thiophen-3-yl)phenyl)thio)aniline (1.3 g, 3.96 mmol, 1.0 eq) and the mixture was stirred at 80°C for 2 hours. The reaction was completed and monitored by LCMS, and the mixture was extracted with DCM and washed with brine. The organic layer was dried with anhydrous Na₂SO₄ and filtered. The filtrate was concentrated and purified by silica gel column chromatography eluted at DCM : MeOH = 10 : 1 to obtain a brown solid of 4-((4-(thiophen-3-yl)phenyl)thio)benzene-1,2-diamine (900 mg) (yield 76%).

¹H NMR (400 MHz, DMSO-d₆) δ 7.77-7.78(m,1H), 7.57-7.76(m,3H), 7.47-7.49 (m,1H), 7.03-7.06 (m,2H), 6.68 (s, 1H), 6.56-6.61 (m, 2H), 4.67-4.84 (m, 4H)

### 1.10.6. Synthesis of methyl (5-((4-(thiophen-3-yl)phenyl)thio)-1H-benzo[d]imidazol-2-yl)carbamate

1,3-bis(methoxycarbonyl)-S-methylisothiourea (76 mg, 0.369 mmol, 1.1 eq) was added in a CH₃COOH (5 mL) solution of 4-((4-(thiophen-3-yl)phenyl)thio)benzene-1,2-diamine (100 mg, 0.335 mmol, 1.0 eq). The reaction mixture was stirred at 80°C for 2 hours. The completion of the reaction was confirmed by LCMS and then the reaction product was extracted with DCM and washed with a saturated solution. The organic layer was washed with saturated NaHCO₃, dried with anhydrous Na₂SO₄ and filtered. The filtrate was concentrated and the material was purified by Prep-TLC to obtain a white solid of methyl (5-((4-(thiophen-3-yl)phenyl)thio)-1H-benzo[d]imidazol-2-yl)carbamate (Chemical Formula 1-10, 50 mg) (yield 39.1%).

¹H NMR (400 MHz, DMSO-d₆):δ ppm 7.82-7.84 (m, 1H), 7.61-7.66 (m, 3H), 7.45-7.54 (m, 3H), 7.10-7.23 (m, 3H), 3.76 (s, 3H)

### 1.11 Synthesis of Chemical Formula 1-11

A synthetic process of a thiobenzimidazole derivative of Chemical Formula 1-11 was shown in Reaction Formula 11 below.

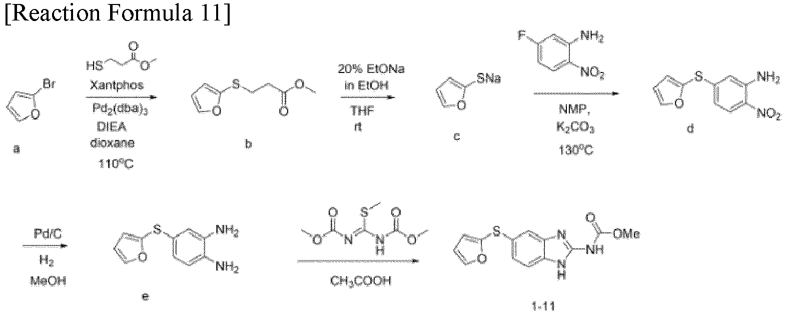

### 1.11.1. Synthesis of Methyl 3-(furan-2-ylthio)propanoate

Methyl 3-mercaptopropanoate (31.6 mL, 285.6 mmol, 7.0 eq), Xantphos (4.787 g, 8.16 mmol, 0.2 eq), DIEA(21.4 mL, 122.4 mmol, 3.0 eq) and Pd₂(dba)₃ (3.623 g, 4.08 mmol, 0.1 eq) were added sequentially to an anhydrous dioxane (50 mL) solution of 2-bromofuran (6.0 g, 40.8 mmol, 1.0 eq). After the addition was completed, the reactant was stirred at 110°C for 16 hours under N₂. The reactant was filtered and the filtrate was concentrated. The obtained product was purified with silica gel (HE : EtOAC = 40 : 1) by column chromatography to obtain a yellow oily liquid of methyl 3-(furan-2-ylthio)propanoate (6.34 g) (yield 83.4%).

### 1.11.2. Synthesis of sodium furan-2-thiolate

20% NaOEt (14.06 g, 41.32 mmol, 1.2 eq) was added at room temperature in a solution of methyl 3-(furan-2-ylthio)propanoate (6.34 g, 34.08 mmol, 1.0 eq) in dry THF (60 mL). The reaction was completed by heating the reaction mixture for 1 hour and then the mixture was concentrated to obtain a brown solid of sodium furan-2-thiolate (4.61 g).

### 1.11.3. Synthesis of 5-(furan-2-ylthio)-2-nitroaniline

An anhydrous N-methylpyrrolidinedimethyl (60 mL) solution of sodium furan-2-thiolate (4.61 g, 37.86 mmol, 1.1 eq), 5-fluoro-2-nitroaniline (5.39 g, 34.4 mmol, 1.0 eq) and K₂CO₃ (14.26 g, 103.2 mmol, 3.0 eq) was stirred at 130°C for 16 hours under N₂. After the reaction was completed, the residue was purified by silica gel column chromatography (HE : EtOAC = 20 : 1) to obtain a yellow solid of 5-(furan-2-ylthio)-2-nitroaniline (860 mg).

### 1.11.4. Synthesis of 4-(furan-2-ylthio)benzene-1,2-diamine

5-(furan-2-ylthio)-2-nitroaniline (860 mg, 3.624 mmol, 1.0 eq) and Pd/C (360 mg) were added to a mixed solvent of MeOH (36 mL) and MeOH/NH₃ (12 mL), and stirred at room temperature for 18 hours in the presence of H₂. The reaction mixture was filtered and the filtrate was concentrated to obtain a purple solid of 4-(furan-2-ylthio)benzene-1,2-diamine (750 mg) (yield 100%).

¹H NMR (400 MHz, CDCl₃): δ ppm 7.49-7.50 (m, 1H), 6.69-6.74 (m, 2H), 6.59-6.62 (m, 2H), 6.39-6.41 (m, 1H), 3.37 (brs, 4H)

### 1.11.5. Synthesis of methyl (5-(furan-2-ylthio)-1H-benzo[d]imidazol-2-yl)carbamate

1,3-bis(methoxycarbonyl)-S-methylisothiourea (100 mg, 0.485 mmol, 1.1 eq) was added in a solution of 4-(furan-2-ylthio)benzene-1,2-diamine (100 mg, 0.485 mmol, 1.0 eq) in CH₃COOH (5 mL) and the mixture was stirred at 80°C for 2 hours. The reactant was monitored by LCMS to confirm that the reaction was completed, and then extracted with DCM and washed with a saturated NaHCO₃ solution. The organic layer was dried with anhydrous Na₂SO₄ and filtered. The filtrate was concentrated and the reaction residue was purified by Prep-TLC to obtain a white solid of methyl (5-(furan-2-ylthio)-1H-benzo[d]imidazol-2-yl)carbamate (Chemical Formula 1-11, 20 mg) (yield 14.2%).

¹H NMR (400 MHz, DMSO-d₆): δ ppm 7.88-7.89 (m, 1H), 7.40 (d, J = 8.4 Hz, 1H), 7.30 (d, J = 8.4 Hz, 1H), 7.07-7.10 (m, 1H), 6.92-6.93 (m, 1H), 6.61-6.63 (m, 1H), 3.78 (s, 3H)

### 1.12 Synthesis of Chemical Formula 1-12

Methyl (5-((3,4-difluorophenyl)thio)-1H-benzo[d]imidazol-2-yl)carbamate was synthesized in the same manner as in Chemical Formula 1-1 in Example 1.1 using 3,4-difluorobenzenethiol as a starting material (Chemical Formula 1-12, yield 69.2%).

¹H NMR (CDCl₃, 400 MHz) 3.76(s, 3H), 6.93(m, 1H), 7.17(m, 2H), 7.19(d, J=2.32 Hz, 1H), 7.23(q, 1H), 7.38(d, J=10.64 Hz), 7.55(s, 1H), 11.53(m, 1H), 12.02(m, 1H)

### 1.13 Synthesis of Chemical Formula 1-13

Methyl (5-((2.4-difluorophenyl)thio)-1H-benzo[d]imidazol-2-yl)carbamate was synthesized in the same manner as in Chemical Formula 1-1 in Example 1.1 using 2,4-difluorobenzenethiol as a starting material (Chemical Formula 1-13, yield 75.4%).

¹H NMR (DMSO-d6, 400 MHz) 3.75(s, 3H), 7.12(m, 3H), 7.33(m, 1H), 7.38(m, 1H), 7.47(s, 1H), 11.73(m, 2H)

### 1.14 Synthesis of Chemical Formula 1-14

Methyl (5-((5-chloropyridin-2-yl)thio)-1H-benzo[d]imidazol-2-yl)carbamate was synthesized in the same manner as in Chemical Formula 1-1 in Example 1.1 using 5-chloropyridine-2-thiol as a starting material (Chemical Formula 1-14, yield 75.2%).

¹H NMR (DMSO-d6, 400 MHz) 3.77(s, 3H), 6.75(d, J=8.68 Hz, 1H), 7.31(d, J=1.68 Hz), 7.53(d, J=8.2 Hz, 1H), 7.64(s, 1H), 7.69(d, J=2.6 Hz), 8.44(s, 1H), 11.96(m, 2H)

### 1.15 Synthesis of Chemical Formula 1-15

A synthetic process of a thiobenzimidazole derivative of Chemical Formula 1-15 was shown in Reaction Formula 15 below.

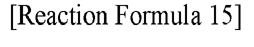

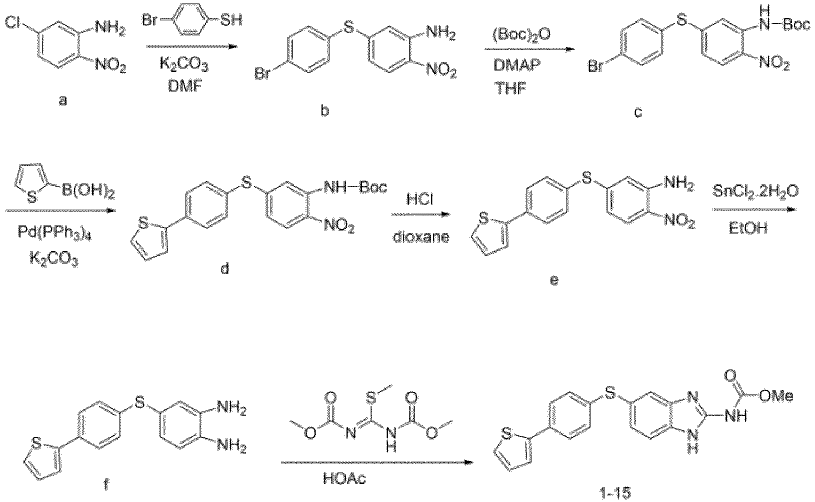

### 1.15.1. Synthesis of 5-((4-Bromophenyl)thio)-2-nitroaniline

Potassium carbonate (1.24 g) was dissolved in a DMF (10 mL) solution of 4-bromobenzenethiol (1.4 g, 8.7 mmol) and added with a DMF (3 mL) solution of 5-chloro-2-nitroaniline (1.49 g, 8.7 mmol) pre-prepared at room temperature. The reaction mixture was stirred and reacted at 90°C for 3 hours, cooled to room temperature, and poured into cold water (500 mL), and then the precipitate was filtered and dried to obtain a yellow solid product of 5-((4-Bromophenyl)thio)-2-nitroaniline (2.8 g) (yield 99%).

### 1.15.2. Synthesis of tert-Butyl (5-((4-bromophenyl)thio)-2-nitrophenyl)carbamate

Di-tert-butyl dicarbonate (5.14 g, 23.5 mmol) and DMAP (0.146 g) were added to a THF (30 mL) solution of 5-((4-Bromophenyl)thio)-2-nitroaniline (2.8 g, 8.7 mmol) and heated, refluxed, and reacted for 1 hour. The completion of the reaction was confirmed by TLC, and the reactant was cooled to room temperature and evaporated under reduced pressure to remove the solvent. The product was dissolved in methanol (30 mL), added with potassium carbonate (5.2 g), stirred at room temperature for 6 hours and then treated after reaction to obtain tert-Butyl (5-((4-bromophenyl)thio)-2-nitrophenyl)carbamate (1.48 g) (yield 40%).

### 1.15.3. Synthesis of tert-butyl (2-nitro-5-((4-(thiophen-2-yl)phenyl)thio)phenyl)carbamate

Thiophen-2-ylboronic acid (0.42 g, 3.28 mmol, 2.0 eq), Tetrakis(triphenylphosphine)palladium (0.18 g, 0.16 mmol, 0.1 eq), and potassium carbonate (0.66 g, 4.80 mmol, 3 eq) were added to a DMF (5 mL) solution of tert-butyl (5-((4-bromophenyl)thio)-2-nitrophenyl)carbamate (0.7 g, 1.6 mmol) and stirred and reacted at 100°C for 15 hours. The mixture was cooled to room temperature, extracted with a methylene chloride solution, washed with a Brine solution once, dried with anhydrous magnesium sulfate and evaporated under reduced pressure. Then, the mixture was separated and purified by silica-Gel column chromatography with an ethyl acetate and hexane (7 : 3, v/v) developer to obtain a light yellow solid product of tert-butyl (2-nitro-5-((4-(thiophen-2-yl)phenyl)thio)phenyl)carbamate (0.48 g) (yield 70%).

### 1.15.4. Synthesis of 2-nitro-5-((4-(thiophen-2-yl)phenyl)thio)aniline

An HCl/dioxane (6 mL) solution of tert-butyl (2-nitro-5-((4-(thiophen-2-yl)phenyl)thio)phenyl)carbamate (0.48 g, 1.1 mmol) was reacted at room temperature for 2 hours, poured into a cold saturated aqueous sodium bicarbonate solution (30 mL), and then extracted with an excess of methylene chloride solution. The extract was dried with anhydrous sodium sulfate, filtered, and evaporated under reduced pressure to remove the solvent, and purified by silica gel column chromatography to obtain 2-nitro-5-((4-(thiophen-2-yl)phenyl)thio)aniline (413 mg) (yield 78%).

### 1.15.5. Synthesis of 4-((4-(thiophen-2-yl)phenyl)thio)benzene-1,2-diamine

Tin(II) chloride dihydrate (1.74 g, 7.7 mmol, 6.4 eq) was added in an ethanol (15 mL) solution of 2-nitro-5-((4-(thiophen-2-yl)phenyl)thio)aniline (413 mg, 1.2 mmol), heated and refluxed for 3 hours, cooled to room temperature, and the added with water (50 mL). The mixture was extracted with an excess of ethyl acetate solvent, and then the solvent was evaporated under reduced pressure to be removed to obtain a solid reaction mixture. The mixture was separated and purified by column chromatography using silica gel using an ethyl acetate and hexane (1 : 3, v/v) solvent to obtain 4-((4-(thiophen-2-yl)phenyl)thio)benzene-1,2-diamine (0.20 g) (yield 57%).

### 1.15.6. Synthesis of Methyl (5-((4-(thiophen-2-yl)phenyl)thio)-1H-benzo[d]imidazol-2-yl)carbamate

4-((4-(thiophen-2-yl)phenyl)thio)benzene-1,2-diamine (0.1 g, 0.33 mmol) and 1,3-bis(methoxycarbonyl)-S-methylisothiourea (0.55 g, 2.6 mmol, 8 eq) were dissolved in 5%-AcOH in Ethanol 3 ml and reacted at 80°C for 2 hours. The completion of the reaction was confirmed by TLC, and the reactant was extracted with an excess of methylene chloride, neutralized with saturated sodium bicarbonate, dried with anhydrous sodium sulfate, and separated by silica gel column chromatography to obtain a light brown solid of methyl (5-((4-(thiophen-2-yl)phenyl)thio)-1H-benzo[d]imidazol-2-yl)carbamate (Chemical Formula 1-15, 90 mg) (yield 72%).

¹H NMR (DMSO-d₆, 400 MHz) 3.76(s, 3H), 7.12(m, 3H), 7.23(d, J=8.16Hz, 1H), 7.47(m, 2H), 7.51-7.58(m, 4H), 11.77(m, 2H)

### 1.16 Synthesis of Chemical Formula 1-16

A synthetic process of a thiobenzimidazole derivative of Chemical Formula 1-16 was shown in Reaction Formula 16 below.

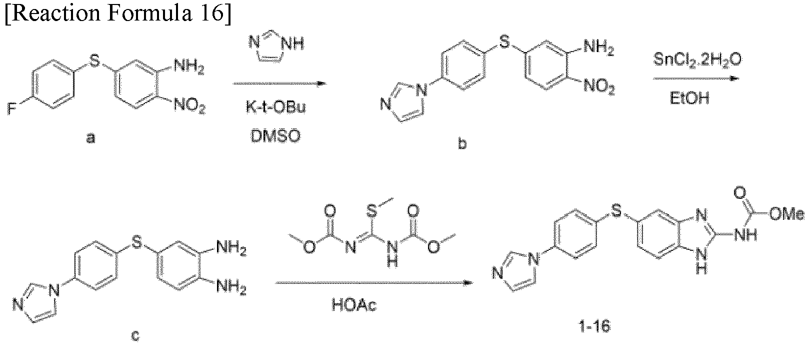

### 1.16.1. Preparation of 5-((4-(1H-imidazole-1-yl)phenylthio)-2-nitroaniline

1H-imidazole (0.23 g, 3.3 mmol) was added to a DMSO (5 mL) solution of 5-((4-fluorophenyl)thio)-2-nitroaniline (0.85 g, 3.2 mmol), stirred for 5 minutes, slowly added with potassium t-butoxide (0.36 g, 3.2 mmol), and then reacted at 90°C for 2 hours. The reaction mixture was extracted with ethyl acetate, washed with water, and evaporated under reduced pressure to remove the solvent and obtain a mixed product. The mixed product was separated and purified by column chromatography using silica gel using an ethyl acetate and hexane (3 : 1, v/v) solvent to obtain 5-((4-(1H-imidazole-1-yl)phenylthio)-2-nitroaniline (0.32 g) (yield 32%).

### 1.16.2. Preparation of 5-((4-(1H-imidazole-1-yl)phenylthio)-1,2-diamine

Tin chloride two hydrate (6 eq) was added in an ethanol (10 mL) solution of 5-((4-(1H-imidazole-1-yl)phenylthio)-2-nitroaniline (0.309 g, 1.0 mmol), heated and refluxed for 4 hours, cooled to room temperature, and then added with water (50 mL). The mixture was extracted with an excess of ethyl acetate solvent, and then the solvent was evaporated under reduced pressure and removed to obtain a solid reaction mixture. The reaction mixture was separated and purified by column chromatography using silica gel using an ethyl acetate and hexane (3 : 1, v/v) solvent to obtain 5-((4-(1H-imidazole-1-yl)phenylthio)-1,2-diamine (0.30 g) (yield 98%).

### 1.16.3. Synthesis of methyl (5-((4-(1H-imidazol-1-yl)phenyl)thio)-1H-benzo[d]imidazol-2-yl)carbamate

1,3-bis(methoxycarbonyl)-2-methyl-2-thiopseudourea (0.52 g, 2.5 mmol) was added to an ethanol (3.0 mL) solution of 5% acetic acid of 5-((4-(1H-imidazole-1-yl)phenylthio)-1,2-diamine (0.28 g, 1.0 mmol) and then heated and refluxed for 5 hours. The reaction mixture was filtered and sufficiently washed with methanol to obtain methyl (5-((4-(1H-imidazol-1-yl)phenyl)thio)-1H-benzo[d]imidazol-2-yl)carbamate (Chemical Formula 1-16, 0.26 g) (yield 70%).

¹H NMR (DMSO-d₆, 400 MHz) 3.76(s, 3H), 7.18(s, 1H), 7.23(m, 3H), 7.48(m, 1H), 7.58(m, 3H), 7.68(s, 1H), 8.20(s, 1H), 11.46(m, 1H), 11.94(m, 1H)

### 1.17 Synthesis of Chemical Formula 1-17

Methyl (5-((4,6-dimethylpyrimidin-2-yl)thio)-1H-benzo[d]imidazol-2-yl)carbamate was synthesized in the same manner as in Chemical Formula 1-1 in Example 1.1 using 4,6-dimethylpyrimidine-2-thiol as a starting material (Chemical Formula 1-17, yield 75.7%).

¹H NMR (DMSO-d6, 400 MHz) 2.25(s, 6H), 3.77(s, 3H), 6.93(s, 1H), 7.26(d, J=8.24 Hz, 1H), 7.45(d, J=8.20 Hz, 1H), 7.61(s, 1H), 11.74(m, 2H)

### 1.18 Synthesis of Chemical Formula 1-18

Methyl (5-(pyridin-2-ylthio)-1H-benzo[d]imidazol-2-yl)carbamate was synthesized in the same manner as in Chemical Formula 1-1 in Example 1.1 using pyridine-2-thiol as a starting material (Chemical Formula 1-18, yield 72.5%).

¹H NMR (DMSO-d6, 400 MHz) 3.77(s, 3H), 6.73(d, J=8.08, 1H), 7.08(m, 1H), 7.28(d, J=6.72 Hz, 1H), 7.55(m, 2H), 7.64(s, 1H), 8.37(d, J=3.36 Hz, 1H), 11.89(m, 2H).

### 1.19 Synthesis of Chemical Formula 1-19

A synthetic process of a thiobenzimidazole derivative of Chemical Formula 1-19 was shown in Reaction Formula 19 below.

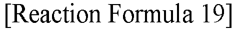

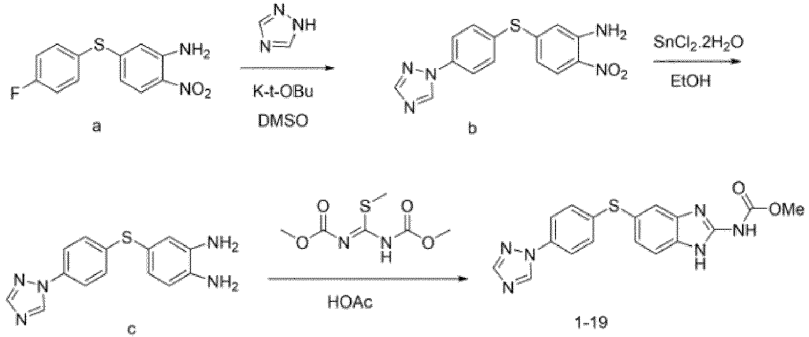

### 1.19.1. Synthesis of 5-((4-(1H-1,2,4-triazol-1-yl)phenyl)thio)-2-nitroaniline

1H-1,2,4-triazole (2.2 g, 32 mmol) was added to a DMSO (30 mL) solution of 5-((4-fluorophenyl)thio)-2-nitroaniline (2.0 g, 29 mmol), stirred for 5 minutes, slowly added with potassium t-butoxide (3.6 g, 33 mmol), and then reacted at 90°C for 4 hours. The reaction mixture was extracted with ethyl acetate, washed with water, and evaporated under reduced pressure to remove the solvent and obtain a mixed product. The mixed product was separated and purified by column chromatography using silica gel using an ethyl acetate and hexane (3:1, v/v) solvent to obtain 5-((4-(1H-1,2,4-triazol-1-yl)phenyl)thio)-2-nitroaniline (0.94 g) (yield 47%).

### 1.19.2. Synthesis of 4-((4-(1H-1,2,4-triazol-1-yl)phenyl)thio)benzene-1,2-diamine

SnCl₂ 2H₂O (6 eq) was added to an ethanol (100 mL) solution of 5-((4-(1H-1,2,4-triazol-1-yl)phenyl)thio)-2-nitroaniline (0.94 g, 3.0 mmol), heated and refluxed for 5 hours, cooled to room temperature, and then added with water (300 mL). The mixture was extracted with an excess of ethyl acetate solvent, and then the solvent was evaporated under reduced pressure and removed to obtain a solid reaction mixture. The reaction mixture was separated and purified by column chromatography using silica gel using an ethyl acetate and hexane (3 : 1, v/v) solvent to obtain 4-((4-(1H-1,2,4-triazol-1-yl)phenyl)thio)benzene-1,2-diamine (0.85 g) (yield 99%).

### 1.19.3. Synthesis of methyl (5-((4-(1H-1,2,4-triazol-1-yl)phenyl)thio)-1H-benzo[d]imidazol-2-yl)carbamate

1,3-bis(methoxycarbonyl)-2-methyl-2-thiopseudourea (1.5 g, 7.7 mmol) was added to an ethanol (10 mL) solution of 5% acetic acid of 4-((4-(1H-1,2,4-triazol-1-yl)phenyl)thio)benzene-1,2-diamine (0.85 g, 3.0 mmol) and then heated and refluxed for 5 hours. The reaction mixture was cooled to room temperature and then filtered. At this time, the reaction mixture was sufficiently washed with methanol to obtain methyl (5-((4-(1H-1,2,4-triazol-1-yl)phenyl)thio)-1H-benzo[d]imidazol-2-yl)carbamate (Chemical Formula 1-19, 0.81 g) (yield 74%).

¹H NMR (DMSO-d₆, 400 MHz) 3.76(s, 3H), 7.25(m, 1H), 7.28(d, J=8.6 Hz, 2H), 7.47(m, 1H), 7.57(s, 1H), 7.78(d, J=8.6 Hz, 2H), 8.21(s, 1H), 9.23(s, 1H), 11.80(m, 2H)

### 1.20 Synthesis of Chemical Formula 1-20

Methyl(5-((4-(4-methyl-1H-imidazol-1-yl)phenyl)thio)-1H-benzo[d]imidazol-2-yl)carbamate was synthesized in the same manner as in Chemical Formula 1-19 in Example 1.19 using 4-methyl-1H-imidazole as a starting material (Chemical Formula 1-20, yield 78.1%).

¹H NMR (DMSO-d6, 400 MHz) 2.14(s, 3H), 3.76(s, 3H), 7.23(d, J=8.4 Hz, 3H), 7.37(s, 1H), 7.47(m, 1H), 7.53(d, J=8.8 Hz, 3H), 8.07(s, 1H), 11.47(m, 1H), 12.01(M, 1H)

### 1.21 Synthesis of Chemical Formula 1-21

Methyl(5-((4-(3,5-dimethyl-1H-1,2,4-triazol-1-yl)phenyl)thio)-1H-benzo[d]imidazol-2-yl)carbamate was synthesized in the same manner as in Chemical Formula 1-19 in Example 1.19 using 3,5-dimethyl-1H-1,2,4-triazole as a starting material (Chemical Formula 1-21, yield 71.9%).

¹H NMR (DMSO-d6, 400 MHz) 2.24(s, 3H), 2.37(s, 3H), 3.76(s, 3H), 7.20(d, J=8.8 Hz, 2H), 7.28(m, 1H), 7.43(d, J=8.8 Hz, 2H), 7.15(m, 1H), 7.60(s, 1H), 11.41(m, 1H), 12.37(m, 1H)

### 1.22 Synthesis of Chemical Formula 1-22

Methyl(5-((4-(3-methyl-1H-1,2,4-triazol-1-yl)phenyl)thio)-1H-benzo[d]imidazol-2-yl)carbamate was synthesized in the same manner as in Chemical Formula 1-19 in Example 1.19 using 3-methyl-1H-1,2,4-triazole as a starting material (Chemical Formula 1-22, yield 70.4%).

¹H NMR (DMSO-d6, 400 MHz) 2.37(s, 3H), 3.76(s, 3H), 7.26(m, 3H), 7.50(m, 1H), 7.56(s, 1H), 7.73(d, J=8.4 Hz, 2H), 9.08(s, 1H), 11.46(m, 1H), 12.04(m, 1H)

### Example 2. Measurement of anticancer activity of thiobenzimidazole derivatives

### 2.1 Confirmation of cell viability of thiobenzimidazole derivatives

A human triple-negative breast cancer (TNBC) cell line MDA-MB-231 (Cell seeding numbers: 1(M231) × 10⁴ cells/wells (confluency ≧ 25%) and an HER2-positive breast cancer (HER2 + BC) cell line JIMT-1 (Cell seeding numbers: 1.2(JIMT) × 10⁴ cells/wells (confluency ≧ 25%) were used in the experiment.

The cell lines were cultured in a Dulbecco's modified Eagle's medium (DMEM) containing 10% fetal bovine serum (FBS), streptomycin-penicillin (100 U/mL) and Fungizone (0.625 µg/mL) in an environment of 5% CO₂ and 37°C, respectively.

In the human breast cancer cell lines MDA-MB-231 and JIMT-1, thiobenzimidazole derivatives of Chemical Formulas 1-1, 1-2, 1-3, 1-5, 1-6, 1-7, 1-8, 1-10, 1-11, 1-12, 1-13, 1-14, 1-15, 1-16, 1-17, 1-19, 1-20, 1-21, and 1-22 were treated at various concentrations of 0, 0.5, 1, and 5 µM for 72 hours, respectively, and then cell viability was measured by an MTS assay. In the MTS assay, the cells were attached to a 96 well plate for 24 hours, treated with the thiobenzimidazole derivatives for 72 hours, and then developed with MTS (3-(4,5-dimethylthiazol-2-yl)-5-(3-carboxymethoxyphenyl)-2-(4-sulfophenyl)-2H-tetrazolium) for 4 hours, and then absorbance was measured at 490 nm using a Spectramax Plus384 microplate analyzer.

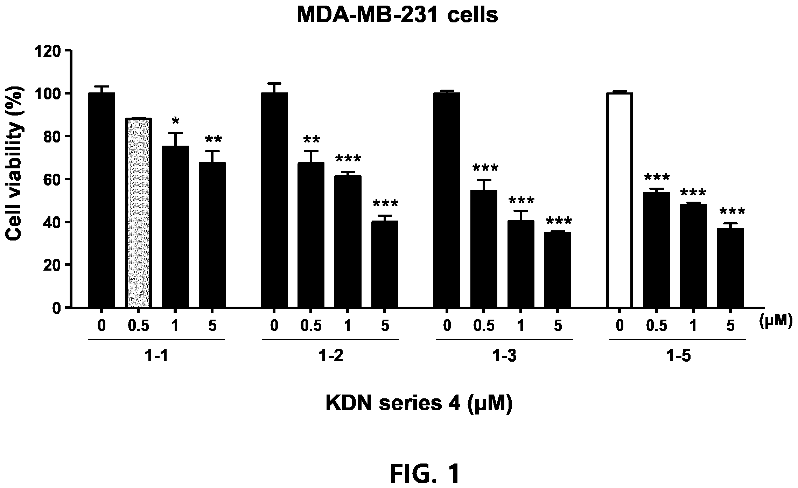

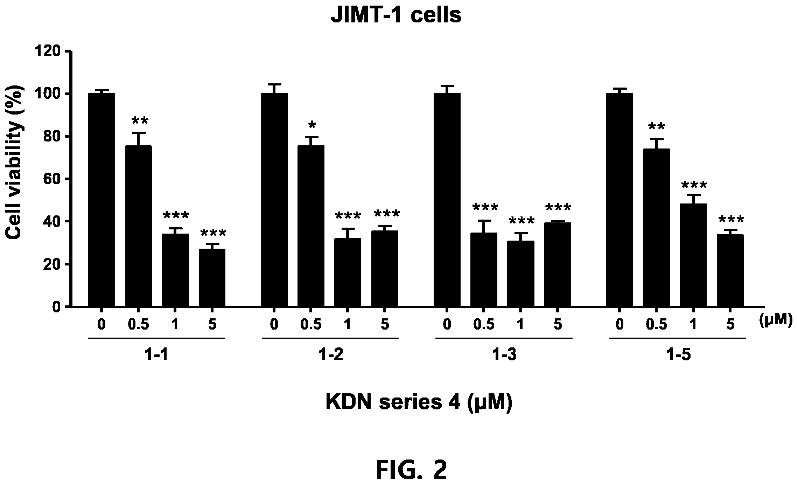

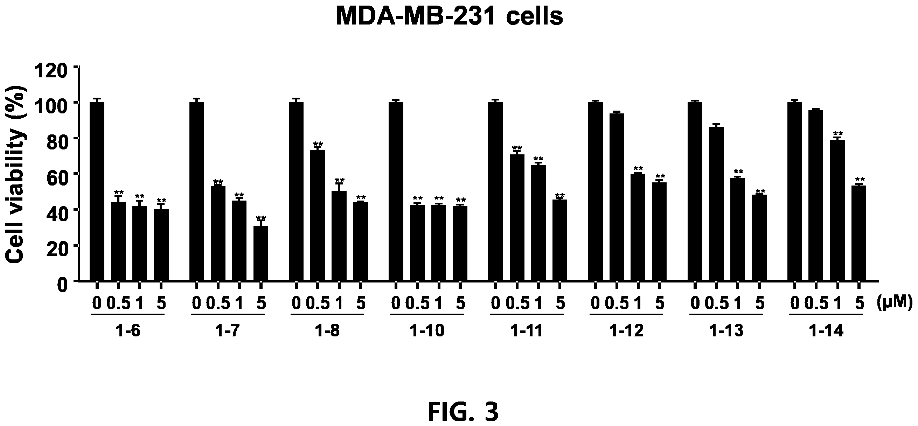

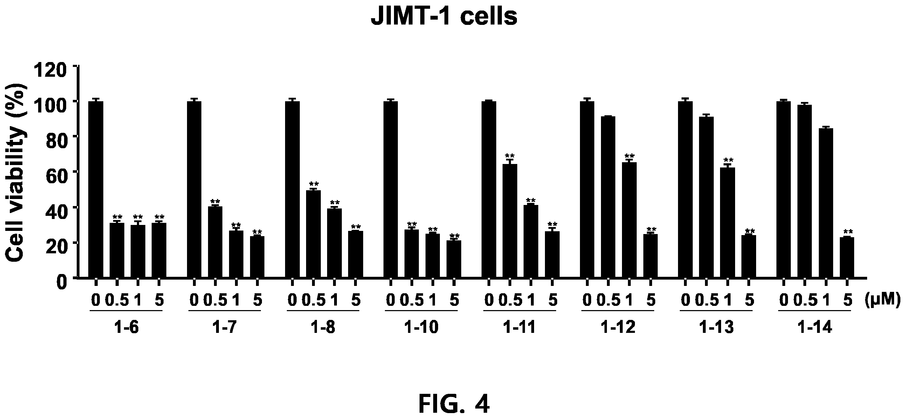

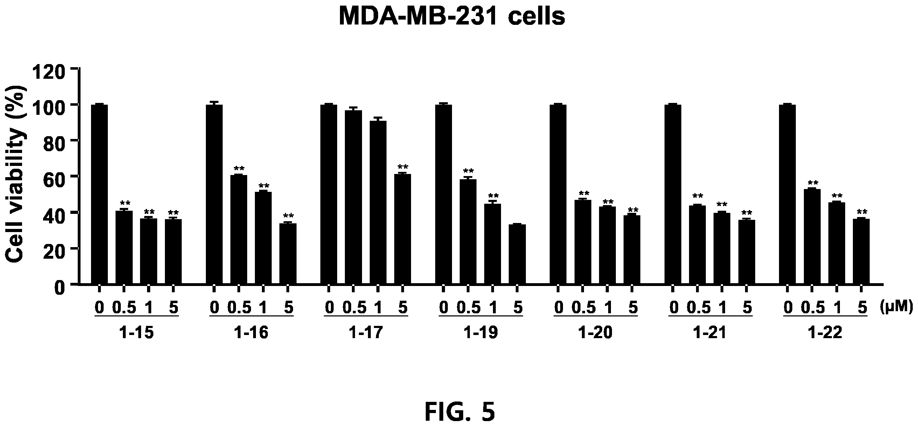

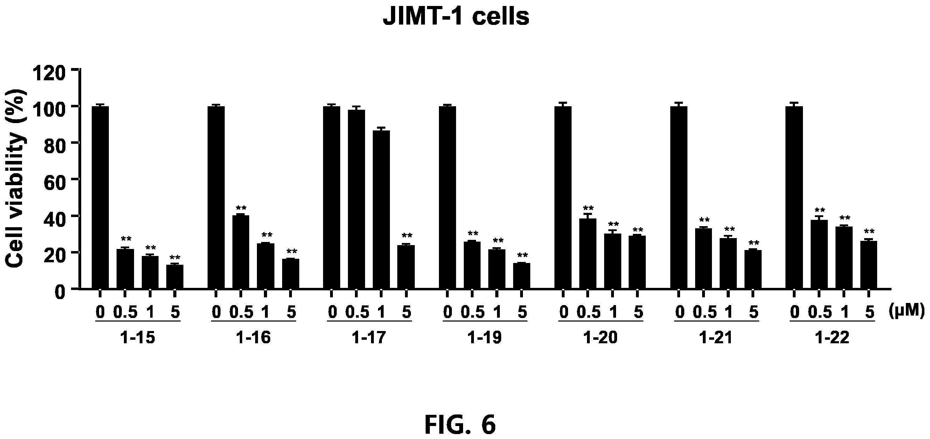

The measured results were illustrated in FIGS. 1 to 6. FIGS. 1, 3, and 5 confirmed the cell viability of the MDA-MB-231 cell line, and FIGS. 2, 4, and 6 confirmed the cell viability of the JIMT-1 cell line.

As a result, it was confirmed that in the human breast cancer cell lines MDA-MB-231 and JIMT-1, most of the thiobenzimidazole derivatives by Chemical Formulas 1-1, 1-2, 1-3, 1-5, 1-6, 1-7, 1-8, 1-10, 1-11, 1-12, 1-13, 1-14, 1-15, 1-16, 1-17, 1-19, 1-20, 1-21, and 1-22 inhibited the cell viability in a concentration-dependent manner.

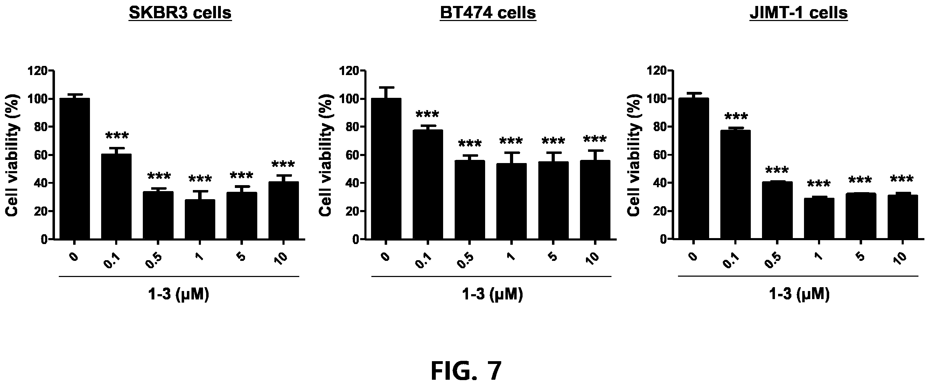

Additionally, HER2-positive breast cancer cell lines SKBR3 and BT474 sensitive to Trastuzumab and an HER2-positive breast cancer cell line JIMT-1 resistant to Trastuzumab were treated with the thiobenzimidazole derivative by Chemical Formula 1-3 in the concentration-dependent manner for 72 hours, and as a result, it was confirmed that the inhibitory effect on cell proliferation was exhibited in all of the three cell lines (FIG. 7).

### 2.2 Confirmation of cell cycle arrest and apoptosis by thiobenzimidazole derivative

The degree of apoptosis of cancer cells induced by the thiobenzimidazole derivative of Chemical Formula 1-3 was measured through DNA content analysis using a flow cytometry. The SKBR3, BT474 and JIMT-1 cell lines were treated with a control (DMSO) and the derivative of Chemical Formula 1-3 at concentrations of 0.1, 0.25 and 0.5 µM for 72 hours, and then the cells were harvested, immobilized with 95% ethanol containing 0.5% Tween-20 for 24 hours, and then stained with propidium iodide (PI, 50 µg/mL) and RNase (50 µg/mL) for 30 minutes. Thereafter, the degree of apoptosis of cancer cells was analyzed using a flow cytometer.

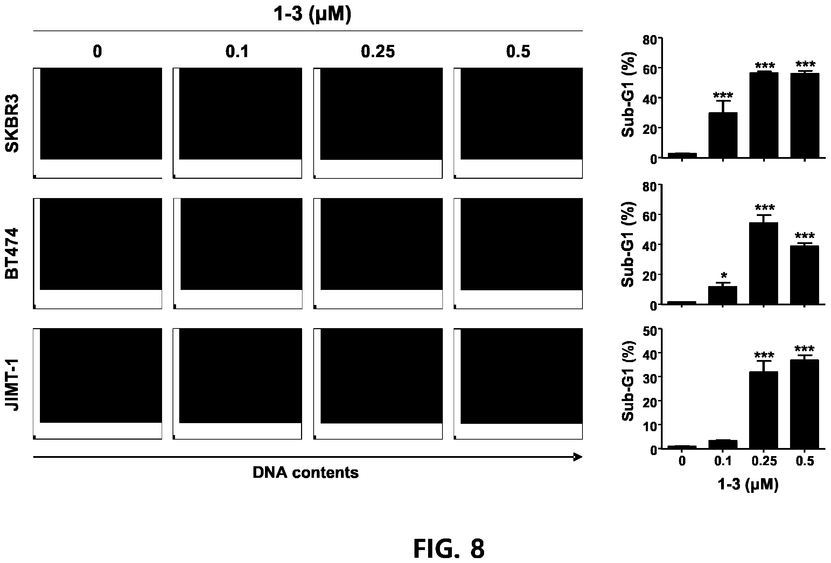

In general, the cell cycle was divided into G1 (cell growth phase)-S (cell replication phase)-G2/M (cell division phase) according to the content of intracellular DNA, and when the apoptosis was induced, the content of intracellular DNA was significantly less than that in the G1 phase by being accompanied by DNA fragmentation. The result of such apoptosis was shown on the cell cycle as a Sub G1 site, and the ratio of Sub G1 was expressed numerically and shown in FIG. 8.

As a result, it was confirmed that the derivative of Chemical Formula 1-3 significantly induced apoptosis (Sub-G1 population) in SKBR3, BT474, and JIMT-1 breast cancer cell lines, and arrested the G2/M phase cell cycle. Apoptosis experiments were independently performed three times, and significance was verified by an unpaired Students t-test (*p < 0.01; DMSO control vs NCT-58 or NCT-407).

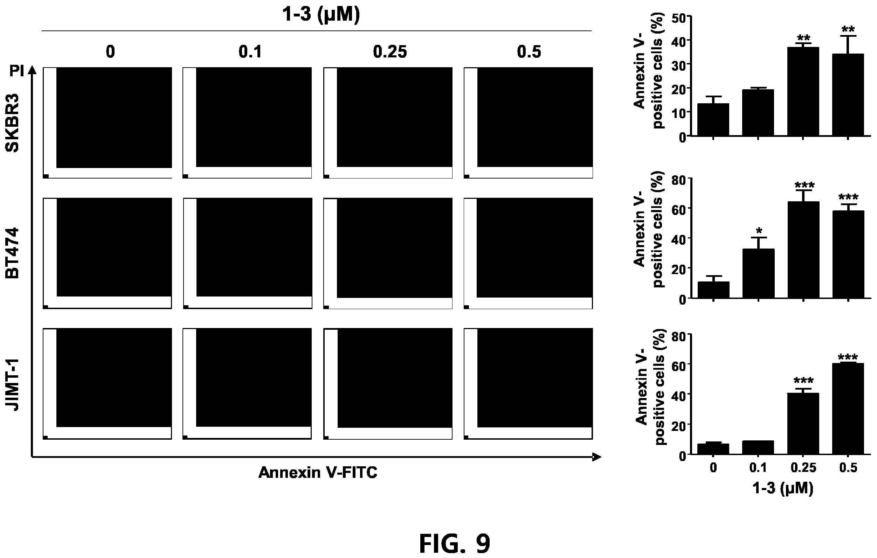

In addition, in order to confirm the apoptosis-inducing effect of the derivative of Chemical Formula 1-3, the control (DMSO) and the derivative of Chemical Formula 1-3 were treated at concentrations of 0.1, 0.25, and 0.5 µM for 72 hours, and then the degree of apoptosis was confirmed by Annexin V/PI staining. As a result, it was confirmed that early and late apoptosis were effectively induced in a concentration-dependent manner by treatment with the derivative of Chemical Formula 1-3 (FIG. 9).

### Example 3. Preparation of hydrochloride of thiobenzimidazole derivative

A synthesis process of Compound 1-23, which was hydrochloride of the thiobenzimidazole derivative of Chemical Formula 1-3, was shown in Reaction Formula 23 below.

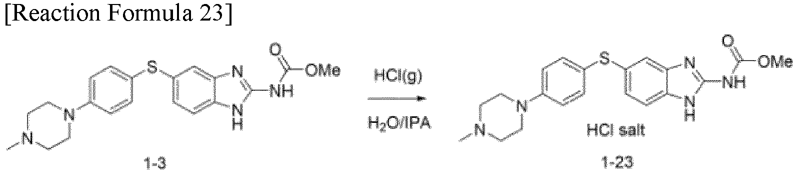

Hydrogen chloride (HCl) gas was injected into a methanol (75 mL) suspension of methyl (5-((4-(4-methylpiperazin-1-yl)phenyl)thio)-1H-benzo[d]imidazol-2-yl)carbamate (370 mg, 0.93 mmol) at 2-minute intervals until the pH became 1 or less. The reactant was evaporated under reduced pressure to obtain an oily product, added with isopropyl alcohol (37 mL) again, dissolved by heating, and then cooled to room temperature to produce a solid suspension. Thereafter, while isopropyl ether (35 mL) was dividedly added to the solid suspension, the mixture was stirred for 3 hours and then filtered. At this time, the mixture was washed with a mixed solvent of IPA and IPE, dried with hot air at 50°C for 1 hour, and dried in vacuum at 40°C for 2 hours to obtain an off-white solid of methyl(5-((4-(4-methylpiperazin-1-yl)phenyl)thio)-1H-benzo[d]imidazol-2-yl)carbamate hydrochloric acid salt (Chemical Formula 1-23, off-white solid, 350 mg) (yield 87%).

¹H NMR (D₂O, 400 MHz) δ 2.93(s, 3H), 3.08-3.14 & 3.19-3.25 (2m, 4H), 3.61(d, J = 12.5 Hz, 2H), 3.64(d, J = 12.5 Hz, 2H), 3.89(s, 3H), 7.04-7.44(m, 7H)

### Example 4. Measurement of anticancer activity of hydrochloride of thiobenzimidazole derivative

### 4.1 Confirmation of cell viability of hydrochloride of thiobenzimidazole derivative

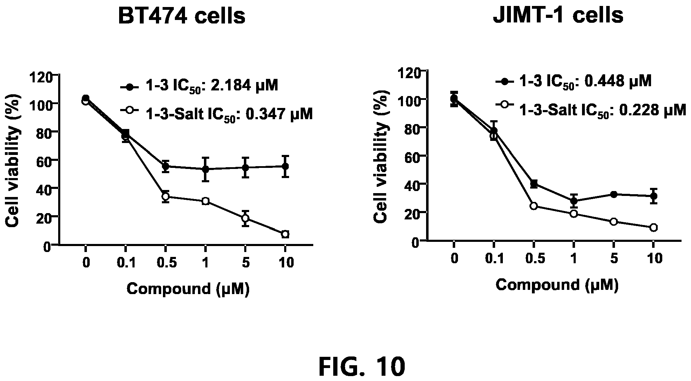

The HER2-positive breast cancer cell line BT474 sensitive to Trastuzumab and the HER2-positive breast cancer cell line JIMT-1 resistant to Trastuzumab were treated with the thiobenzimidazole derivative of Chemical Formula 1-3 prepared above and its hydrochloride (Compound 1-23) at the same concentrations of 1, 0.1, 0.5, 1, 5, and 10 µM for 72 hours, and then cell viability and IC₅₀ were measured by an MTS assay. As a result, in the BT474 cell line, the IC₅₀ of the derivative of Chemical Formula 1-3 was 2.184 µM, and the IC₅₀ of the hydrochloride (Compound 1-23) was 0.347 µM, which was at least 6 times lower there than, and in the JIMT-1 cell line, the IC₅₀ of the derivative of Chemical Formula 1-3 was 0.448 µM, and the IC₅₀ of the hydrochloride (Compound 1-23) was 0.228 µM, which was almost half of the value (FIG. 10).

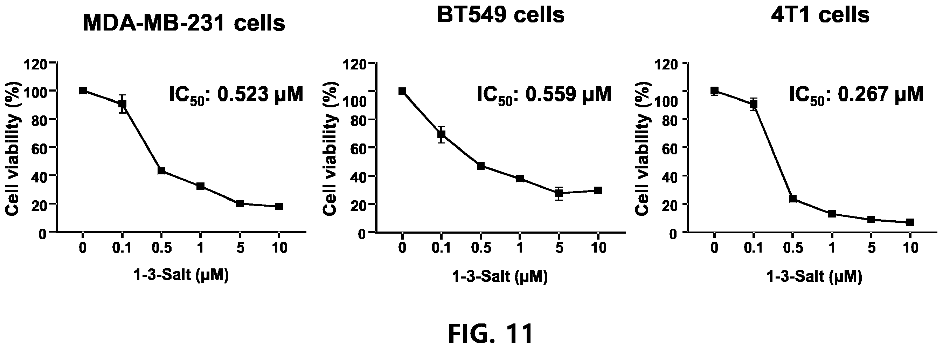

In addition, the triple-negative breast cancer cell lines MDA-MB-231, BT549, and 4T1 were treated with the hydrochloride (Compound 1-23) of the thiobenzimidazole derivative of Chemical Formula 1-3 prepared above for each of concentrations 0, 0.1, 0.5, 1, 5, and 10 µM for 72 hours, and then cell viability and IC₅₀ were measured by an MTS assay. As a result, the three cell lines showed the low IC₅₀ of 0.523 µM, 0.559 µM, and 0.267 µM, respectively (FIG. 11).

The results suggest that the hydrochloride (Compound 1-23) has more improved solubility to exhibit greater inhibition ability of cell viability than the derivative of Chemical Formula 1-3.

### 4.2 Confirmation of cell cycle arrest of hydrochloride of thiobenzimidazole derivative

The HER2-positive breast cancer cell line JIMT-1 and the triple-negative breast cancer cell line MDA-MB-231 were treated with the hydrochloride (Compound 1-23) of the thiobenzimidazole derivative of Chemical Formula 1-3 for each of concentrations 0, 0.1, 0.25, and 0.5 µM for 72 hours, respectively, and then western blot was performed.

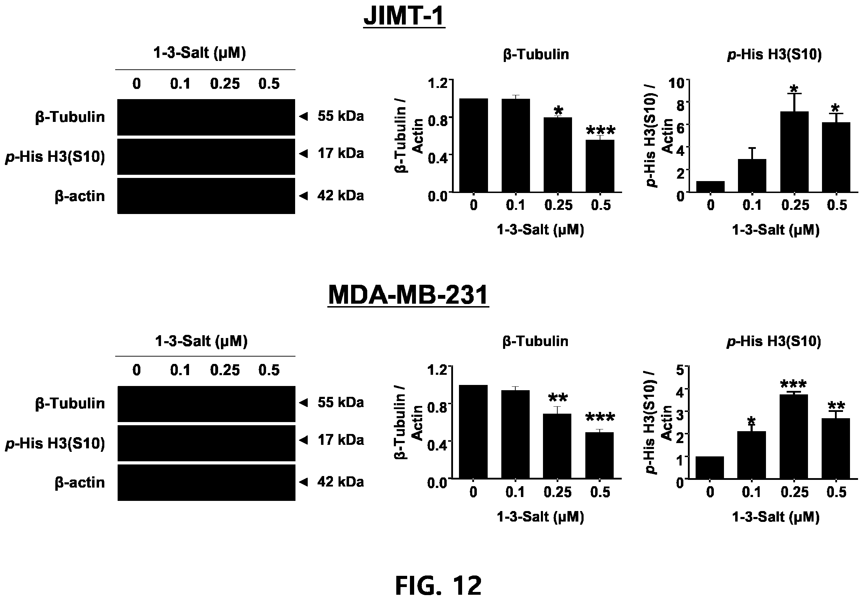

As a result, it was confirmed that the hydrochloride of the present disclosure reduced β-tubulin, a main target protein of a benzimidazole-based anthelmintic, and increased p-Histone H3 (S10), a G2/M cell cycle marker, according to the inhibition of tubulin synthesis (FIG. 12).

### 4.3 Confirmation of anticancer activity of hydrochloride of thiobenzimidazole derivative in other cancer cell lines

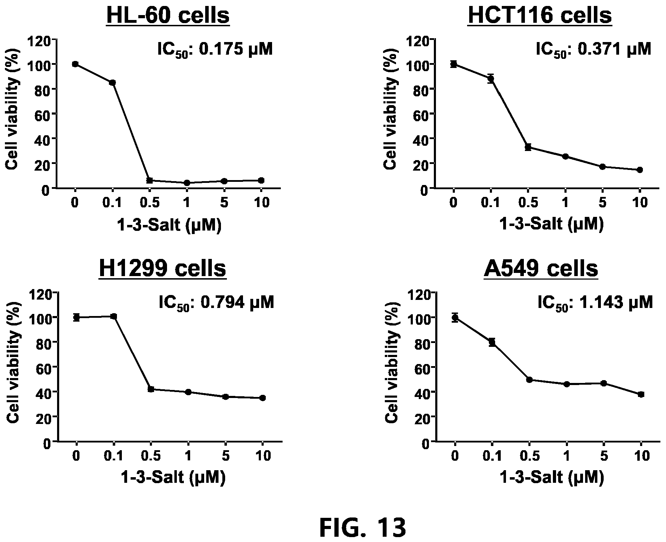

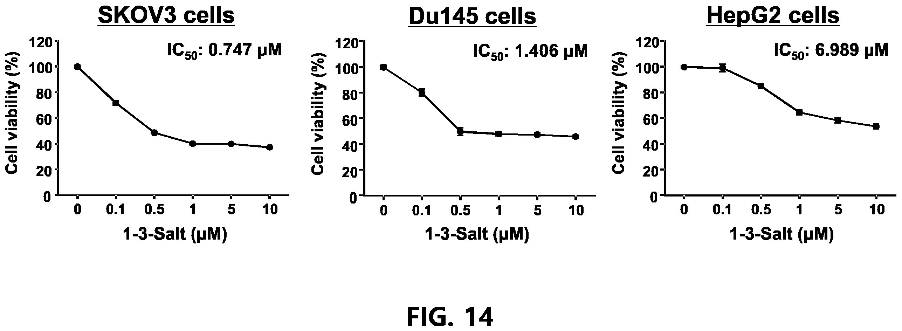

In addition to the breast cancer cell lines, a blood cancer cell line HL-60, a colorectal cancer cell line HCT116, a non-small cell lung cancer cell lines H1299 and A549, an ovarian cancer cell line SKOV3, a prostate cancer cell line Du145, and a liver cancer cell line HepG2 were treated with the hydrochloride (Compound 1-23) of the thiobenzimidazole derivative of Chemical Formula 1-3 for each of concentrations 0, 0.1, 0.25, and 0.5 µM for 72 hours, respectively. Thereafter, as a result of measuring the cell viability and IC₅₀, it was confirmed that there was an excellent cell viability inhibitory effect even in other cancer cell lines (FIGS. 13 and 14).

As described above, although the embodiments have been described by the restricted drawings, various modifications and variations may be applied on the basis of the embodiments by those skilled in the art. For example, even if the described techniques are performed in a different order from the described method, and/or components such as a system, a structure, a device, and a circuit described above are coupled or combined in a different form from the described method, or replaced or substituted by other components or equivalents, an appropriate result may be achieved.

Therefore, other implementations, other embodiments, and equivalents to the appended claims fall within the scope of the claims to be described below.

### Industrial Applicability

The present disclosure relates to a thiobenzimidazole derivative or a pharmaceutically acceptable salt thereof, and a composition for preventing or treating cancer including the derivative as an active ingredient. The thiobenzimidazole derivative of the present disclosure or the pharmaceutically acceptable salt thereof is activated in cancer cells to inhibit tubulin polymerization and exhibits cell toxicity by blocking the cell cycle of a cancer cell and inducing apoptosis when administered to an individual, and thus, may be used for prevention or treatment of cancer, desirably for prevention or treatment of triple-negative breast cancer.

## Claims

1. A thiobenzimidazole derivative represented by Chemical Formula 1 below or a pharmaceutically acceptable salt thereof: 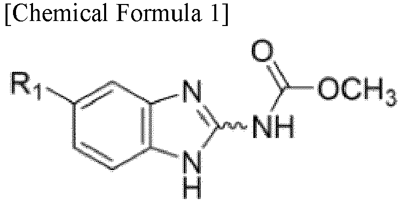 in Chemical Formula 1,
R₁ is -S-R₂ or -S-S-R₂, and
R₂ is any one selected from the group consisting of a substituted or unsubstituted C₃ to C₂₀ aryl group, a substituted or unsubstituted C₃ to C₂₀ heteroaryl group, and a substituted or unsubstituted C₃ to C₂₀ alkylaryl group.

2. The thiobenzimidazole derivative or the pharmaceutically acceptable salt thereof of claim 1, wherein in Chemical Formula 1, the substituted aryl group, heteroaryl group, or alkylaryl group is substituted with at least one selected from the group consisting of a substituted or unsubstituted C₃ to C₁₂ heterocycloalkyl group, a substituted or unsubstituted C₃ to C₁₂ heteroaryl group, and a halogen group.

3. The thiobenzimidazole derivative or the pharmaceutically acceptable salt thereof of claim 1, wherein the thiobenzimidazole derivative represented by Chemical Formula 1 above is any one selected from the group consisting of the following compounds: 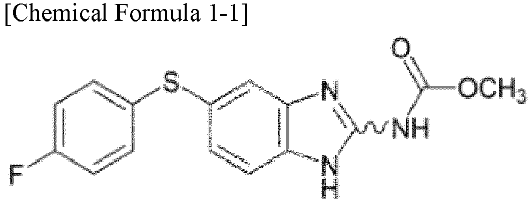 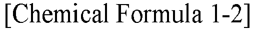 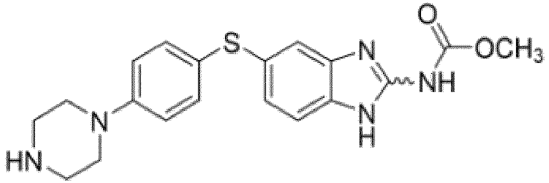 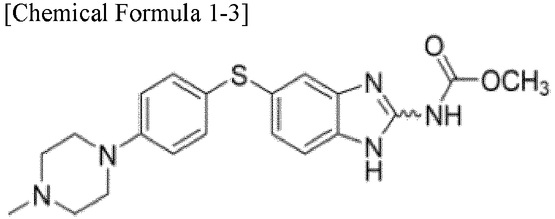 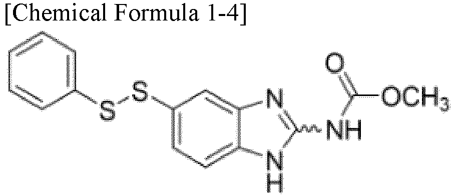 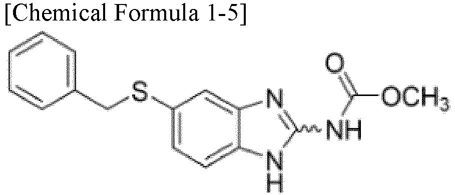 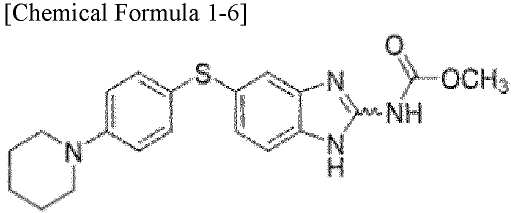 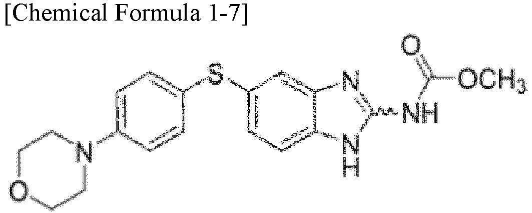 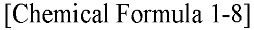 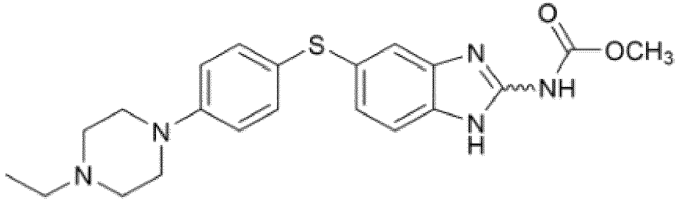 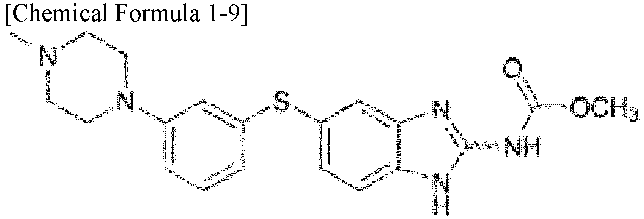 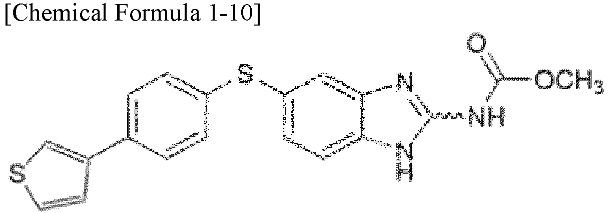 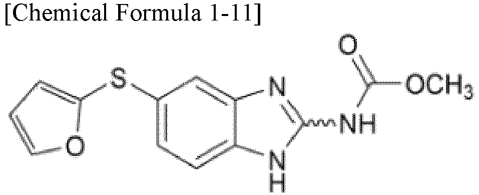 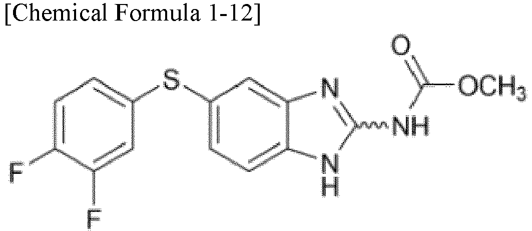 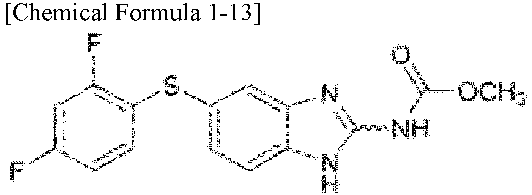 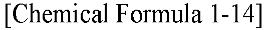 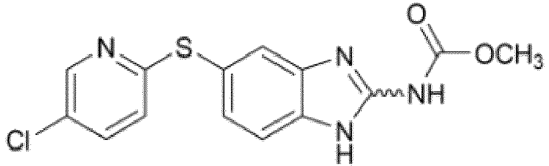 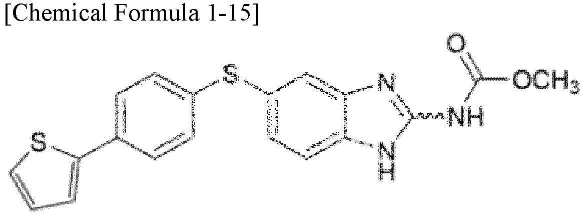 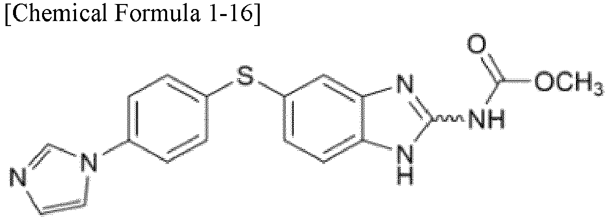 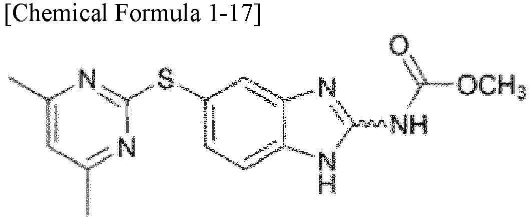 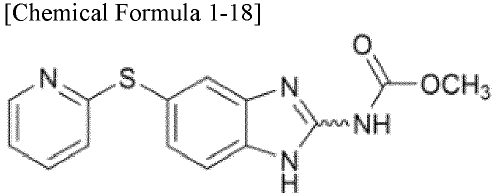 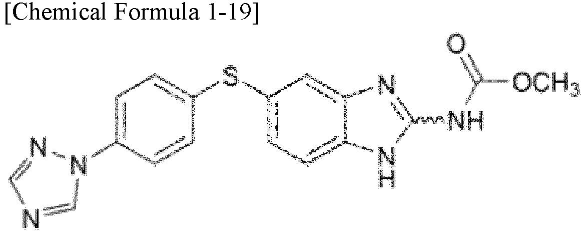 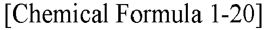 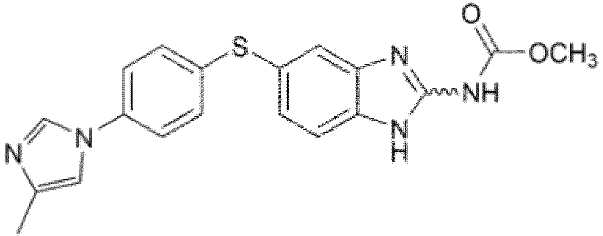 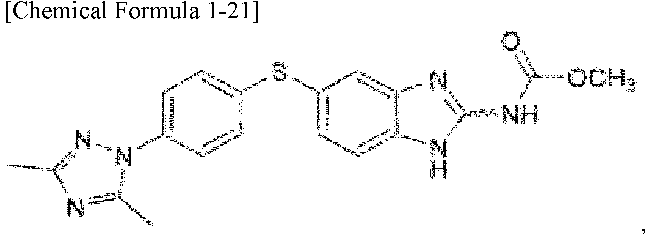 and

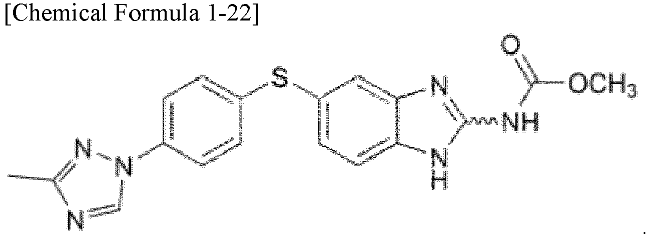

4. The thiobenzimidazole derivative or the pharmaceutically acceptable salt thereof of claim 1, wherein the thiobenzimidazole derivative represented by Chemical Formula 1 above is any one selected from the group consisting of the following compounds: 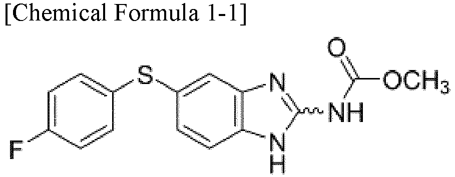 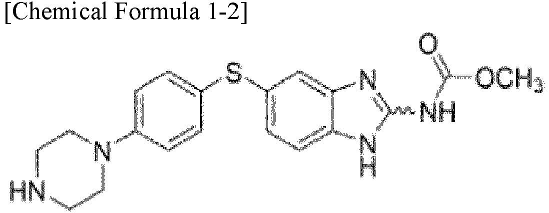 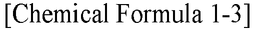 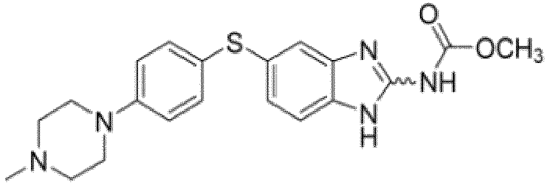 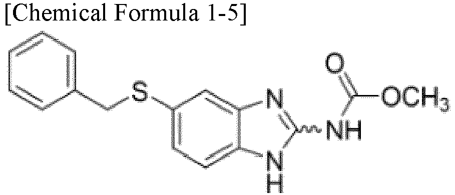 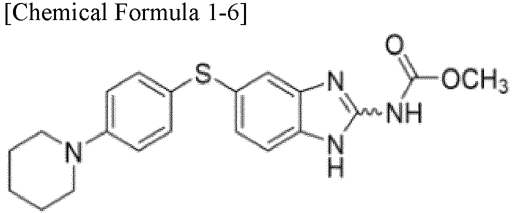 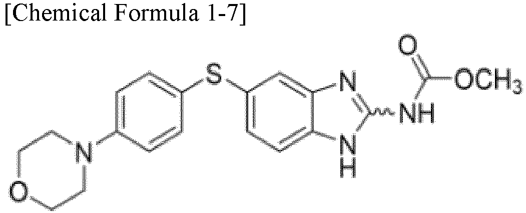 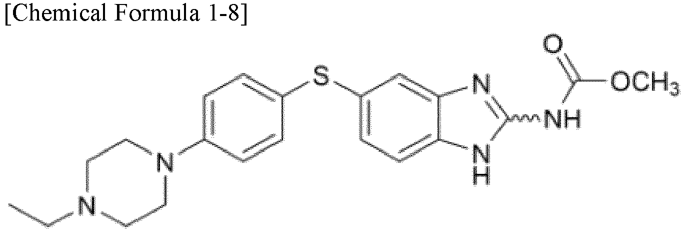 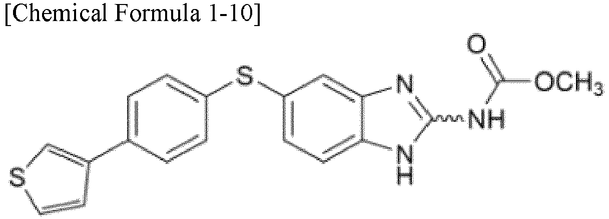 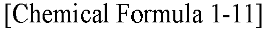 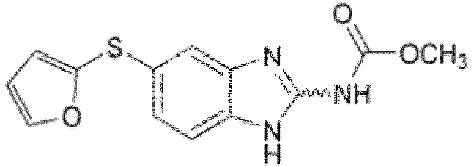 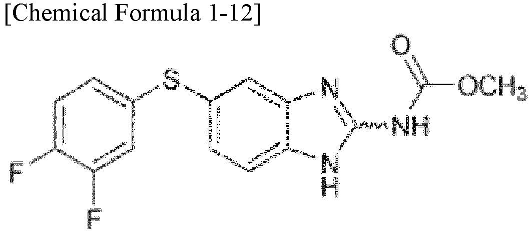 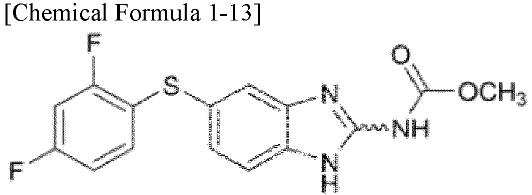 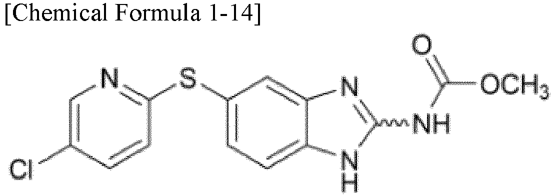 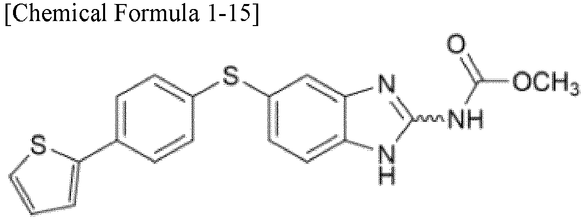 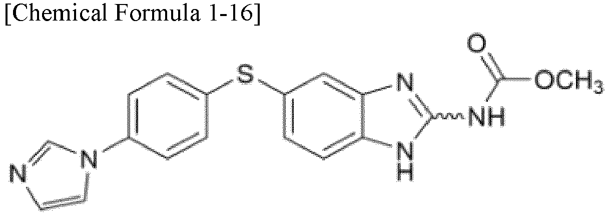 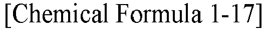 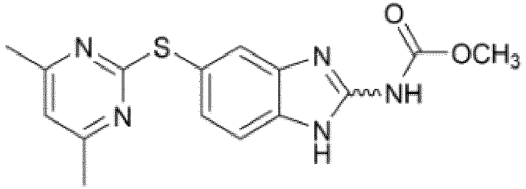 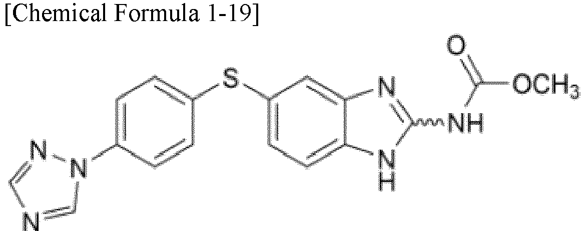 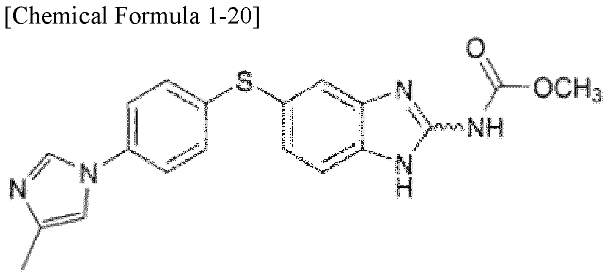 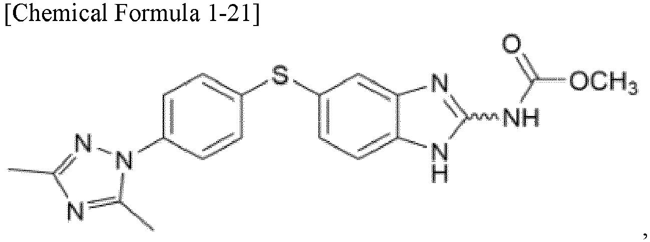 and

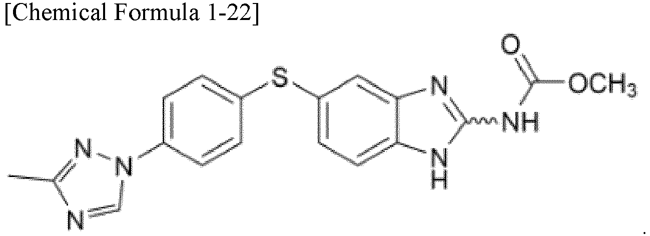

5. The thiobenzimidazole derivative or the pharmaceutically acceptable salt thereof of claim 1, wherein the thiobenzimidazole derivative inhibits tubulin polymerization.

6. The thiobenzimidazole derivative or the pharmaceutically acceptable salt thereof of claim 1, wherein the pharmaceutically acceptable salt of the thiobenzimidazole derivative is at least one selected from the group consisting of hydrochloride, bromate, sulfate, phosphate, nitrate, citrate, acetate, lactate, tartrate, maleate, gluconate, succinate, formate, trifluoroacetate, oxalate, fumarate, glutarate, adipate, methanesulfonate, benzenesulfonate, p-toluenesulfonate, camphorsulfonate, sodium salt, potassium salt, lithium salt, calcium salt, and magnesium salt.

7. The thiobenzimidazole derivative or the pharmaceutically acceptable salt thereof of claim 1, wherein the pharmaceutically acceptable salt of the thiobenzimidazole derivative is hydrochloride.

8. A method for preparing hydrochloride of thiobenzimidazole, the method comprising steps of:
(1) preparing a suspension of a thiobenzimidazole derivative represented by Chemical Formula 1 below; 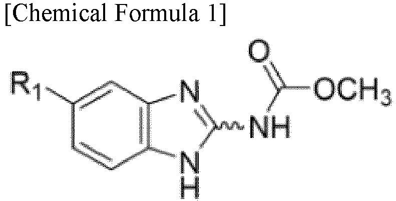
(2) injecting hydrogen chloride (HCl) into the suspension and evaporating under reduced pressure;
(3) adding isopropyl alcohol to the product of step (2) and cooling after heating; and
(4) adding, stirring, and then filtering isopropyl ether to the product of step (3), wherein in Chemical Formula 1,
R₁ is -S-R₂ or -S-S-R₂, and
R₂ is any one selected from the group consisting of a substituted or unsubstituted C₃ to C₂₀ aryl group, a substituted or unsubstituted C₃ to C₂₀ heteroaryl group, and a substituted or unsubstituted C₃ to C₂₀ alkylaryl group.

9. A pharmaceutical composition for preventing or treating cancer, comprising comprising the thiobenzimidazole derivative or the pharmaceutically acceptable salt thereof according to any one of claims 1 to 7 as an active ingredient.

10. The pharmaceutical composition for preventing or treating cancer of claim 9, wherein the pharmaceutical composition induces apoptosis by arresting the cancer cell cycle (cell cycle arrest).

11. The pharmaceutical composition for preventing or treating cancer of claim 9, wherein the cancer is at least one selected from the group consisting of skin cancer, breast cancer, cervical cancer, esophageal cancer, stomach cancer, brain tumor, colon cancer, rectal cancer, colorectal cancer, lung cancer, ovarian cancer, cervical cancer, endometrial cancer, vulvar cancer, kidney cancer, blood cancer, pancreatic cancer, prostate cancer, testicular cancer, larynx cancer, head and neck cancer, thyroid cancer, liver cancer, bladder cancer, osteosarcoma, lymphoma, blood cancer, thymus cancer, urethral cancer, and bronchial cancer.

12. The pharmaceutical composition for preventing or treating cancer of claim 9, wherein the cancer is triple-negative breast cancer.

13. A method for preventing or treating cancer, the method comprising administering the thiobenzimidazole derivative or the pharmaceutically acceptable salt thereof according to any one of claims 1 to 7 to a subject.

14. The method for preventing or treating cancer of claim 13, wherein the benzimidazole derivative or the pharmaceutically acceptable salt thereof induces apoptosis by arresting the cancer cell cycle.

15. The method for preventing or treating cancer of claim 13, wherein the cancer is at least one selected from the group consisting of skin cancer, breast cancer, cervical cancer, esophageal cancer, stomach cancer, brain tumor, colon cancer, rectal cancer, colorectal cancer, lung cancer, ovarian cancer, cervical cancer, endometrial cancer, vulvar cancer, kidney cancer, blood cancer, pancreatic cancer, prostate cancer, testicular cancer, larynx cancer, head and neck cancer, thyroid cancer, liver cancer, bladder cancer, osteosarcoma, lymphoma, blood cancer, thymus cancer, urethral cancer, and bronchial cancer.

16. The method for preventing or treating cancer of claim 13, wherein the cancer is triple-negative breast cancer.
